(19) Europäisches Patentamt
European Patent Office
Office européen des brevets

(11) EP 3 045 104 A1

(12) EUROPEAN PATENT APPLICATION
published in accordance with Art. 153(4) EPC

(43) Date of publication:
**20.07.2016 Bulletin 2016/29**

(21) Application number: **13893454.2**

(22) Date of filing: **13.09.2013**

(51) Int Cl.:
***A61B 1/04*** *(2006.01)*

(86) International application number:
**PCT/JP2013/074903**

(87) International publication number:
**WO 2015/037141 (19.03.2015 Gazette 2015/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Olympus Corporation**
**Shibuya-ku**
**Tokyo 151-0072 (JP)**

(72) Inventors:
- **KAMIYAMA, Toshiya**
  **Tokyo 151-0072 (JP)**
- **KANDA, Yamato**
  **Tokyo 151-0072 (JP)**
- **KITAMURA, Makoto**
  **Tokyo 151-0072 (JP)**
- **KONO, Takashi**
  **Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

# (54) IMAGE PROCESSING DEVICE, METHOD, AND PROGRAM

(57) Provided is an image processing apparatus, a method of processing an image, and an image processing program, which can clearly distinguish an abnormal portion protruding from a surface of a mucous membrane and a bubble, and can accurately detect the abnormal portion. An image processing apparatus 1 includes a contour extracting unit 110 that extracts a plurality of contour pixels from the image acquired by capturing an inside of a lumen of a living body, a feature data calculator 130 that calculates feature data based on pixel values of the plurality of contour pixels and positional relationship among the plurality of contour pixels, and an abnormal portion detector 140 that detects the abnormal portion based on the feature data.

FIG.1

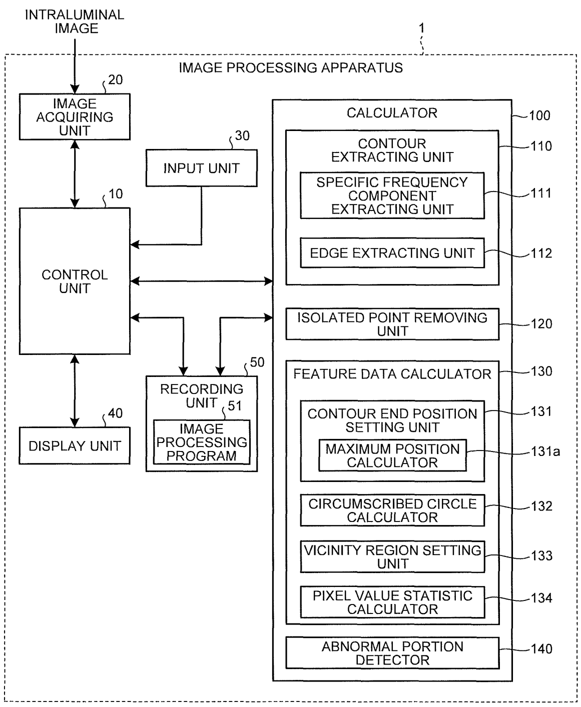


EP 3 045 104 A1

## Description

Field

The present invention relates to an image processing apparatus, a method of processing an image, and an image processing program, for detecting an abnormal portion from an image obtained by capturing an inside of a lumen of a living body.

Background

As a technology related to image processing for an image (hereinafter, referred to as intraluminal image or simply referred to as image) obtained by capturing an inside of a lumen of a living body with a medical observation apparatus such as an endoscope or a capsule endoscope, Patent Literature 1 discloses a technology for detecting an abnormal portion (a lesion-existence candidate image) of a fine structure of a surface of a mucous membrane or a blood vessel running state from the intraluminal image. To be specific, in Patent Literature 1, feature data is calculated from an image of a G (green) component that includes information related to the fine structure of a mucous membrane or the blood vessel image, and existence/non-existence of an abnormal finding is determined using the feature data and a linear discriminant function. As the feature data, for example, shape feature data (an area, a groove width, a peripheral length, circularity, a branching point, an end point, or a branch rate: see Patent Literature 2) of a region extracted by binarization of an image of a specific space frequency component, or feature data (see Patent Literature 3) by a space frequency analysis using a Gabor filter is used. Further, the linear discriminant function is created using feature data calculated from an image of normal and abnormal findings as teacher data.

Citation List

Patent Literatures

Patent Literature 1: Japanese Laid-open Patent Publication No. 2005-192880
Patent Literature 2: Japanese Patent No. 2918162
Patent Literature 3: Japanese Laid-open Patent Publication No. 2002-165757

Summary

Technical Problem

However, when the technology disclosed in Patent Literature 1 is applied to detect an abnormal portion protruding from a surface of a mucous membrane, such as an enlarged fur (swelling) or polyp, an object having a feature similar to the swelling, to be specific, a bubble having a circular edge may be wrongly detected.

The present invention has been made in view of the foregoing, and an objective is to provide an image processing apparatus, a method of processing an image, and an image processing program, which can clearly distinguish the abnormal portion protruding from the surface of the mucous membrane from the bubble, and can accurately detect the abnormal portion.

Solution to Problem

To solve the above-described problem and achieve the object, an image processing apparatus according to the present invention includes: a contour extracting unit configured to extract a plurality of contour pixels from an image acquired by capturing an inside of a lumen of a living body; a feature data calculating unit configured to calculate feature data based on pixel values of the plurality of contour pixels and positional relationship among the plurality of contour pixels; and an abnormal portion detecting unit configured to detect an abnormal portion in the lumen based on the feature data.

A method of processing an image according to the present invention includes: a contour extracting step of extracting a plurality of contour pixels from an image obtained by capturing an inside of a lumen of a living body; a feature data calculating step of calculating feature data based on pixel values of the plurality of contour pixels and positional relationship among the plurality of contour pixels; and an abnormal portion detecting step of detecting an abnormal portion based on the feature data.

An image processing program according to the present invention causes a computer to execute: a contour extracting step of extracting a plurality of contour pixels from an image obtained by capturing an inside of a lumen of a living body; a feature data calculating step of calculating feature data based on pixel values of the plurality of contour pixels and positional relationship among the plurality of contour pixels; and an abnormal portion detecting step of detecting an abnormal portion based on the feature data.

Advantageous Effects of Invention

According to the present invention, an abnormal portion is detected based on feature data based on pixel values of a plurality of contour pixels extracted from an intraluminal image and positional relationship. Therefore, the abnormal portion protruding from a surface of a mucous membrane and a bubble can be clearly distinguished, and the abnormal portion can be accurately detected.

Brief Description of Drawings

FIG. 1 is a block diagram illustrating a configuration of an image processing apparatus according to a first embodiment of the present invention.
FIG. 2 is a schematic diagram illustrating features of a swelling that is an abnormal portion.
FIG. 3 is a schematic diagram illustrating features of a bubble.
FIG. 4 is a flowchart illustrating an operation of the image processing apparatus illustrated in FIG. 1.
FIG. 5 is a flowchart illustrating processing executed by a specific frequency component extracting unit illustrated in FIG. 1.
FIG. 6 is a flowchart illustrating processing executed by an isolated point removing unit illustrated in FIG. 1.
FIG. 7 is a schematic diagram illustrating a creation example of a label ring image.
FIG. 8 is a flowchart illustrating processing executed by a contour end position setting unit illustrated in FIG. 1.
FIG. 9 is a schematic diagram for describing processing of setting an end region.
FIG. 10 is a flowchart illustrating processing executed by a circumscribed circle calculator illustrated in FIG. 1.
FIG. 11 is a schematic diagram for describing processing of calculating center coordinates of a circumscribed circle.
FIG. 12 is a flowchart illustrating processing executed by a vicinity region setting unit illustrated in FIG. 1.
FIG. 13 is a schematic diagram for describing processing of acquiring a vicinity region.
FIG. 14 is a schematic diagram for describing processing of acquiring a vicinity region.
FIG. 15 is a flowchart illustrating processing of creating a specific frequency component image in a modification 1-1.
FIG. 16 is a block diagram illustrating a configuration of an image processing apparatus according to a second embodiment of the present invention.
FIG. 17 is a diagram for describing a go-around profile of an abnormal portion in a circular contour.
FIG. 18 is a flowchart illustrating an operation of an image processing apparatus illustrated in FIG. 16.
FIG. 19 is a flowchart illustrating processing executed by a circular-shaped contour extracting unit illustrated in FIG. 16.
FIG. 20 is a flowchart illustrating processing executed by a maximum-value minimum-value position calculator illustrated in FIG. 16.
FIG. 21 is a diagram for describing an angle calculated by an angle calculator illustrated in FIG. 16, as feature data.
FIG. 22 is a block diagram illustrating a configuration of an image processing apparatus according to a third embodiment of the present invention.
FIG. 23 is a schematic diagram for describing features of a pixel value on a circular contour in a swelling as an abnormal portion.
FIG. 24 is a schematic diagram for describing features of a pixel value on a circular contour in a bubble.
FIG. 25 is a flowchart illustrating an operation of an image processing apparatus illustrated in FIG. 22.
FIG. 26 is a flowchart illustrating processing executed by a facing position pixel correlation value calculator illustrated in FIG. 22.
FIG. 27 is a schematic diagram for describing processing of calculating a correlation value of pixel values between mutually facing pixels.
FIG. 28 is a diagram illustrating a multidimensional space having respective pixel values of the mutually facing pixels as components.

Description of Embodiments

Hereinafter, an image processing apparatus, a method of processing an image, and an image processing program according to embodiments of the present invention will be described with reference to the drawings. Note that the present invention is not limited by these embodiments. Further, the same portion is denoted with the same reference sign in the illustration of the drawings.

(First Embodiment)

FIG. 1 is a block diagram illustrating an image processing apparatus according to a first embodiment of the present invention. An image processing apparatus 1 according to the present first embodiment is a device that applies image processing of detecting an abnormal portion protruding from a surface of a mucous membrane to an intraluminal image (hereinafter, simply referred to as image) acquired by capturing an inside of a lumen of a living body with an endoscope or a capsule endoscope (hereinafter, these endoscopes are simply and collectively referred to as endoscope), as an example. The intraluminal image is typically a color image having predetermined (256 gradations, for example) pixel levels for wavelength components (color components) of R (red), G (green), and B (blue) in each pixel position.

As illustrated in FIG. 1, an image processing apparatus 1 includes a control unit 10 that controls an operation of the entire image processing apparatus 1, an image acquiring unit 20 that acquires image data corresponding to an image captured by the endoscope, an input unit 30 that receives an input signal input from an outside, a display unit 40 that performed various types of display, a recording unit 50 that stores the image data acquired by the image acquiring unit 20 and various programs, and a calculator 100 that executes predetermined image processing for the image data.

The control unit 10 is realized by hardware such as a CPU, and performs instructions to respective units that configure the image processing apparatus 1 and transfers data, and comprehensively controls the operation of the entire image processing apparatus 1, according to the image data input from the image acquiring unit 20 and operation signals input from the input unit 30, by reading various programs recorded in the recording unit 50.

The image acquiring unit 20 is appropriately configured according to a form of a system that includes the endoscope. For example, when a portable recording medium is used to transfer the image data to/from the capsule endoscope, the image acquiring unit 20 is configured from a reader device to which the recording medium is detachably attached, and which reads the recorded image data of an image. When a server that stores the image data of an image captured by the endoscope is installed, the image acquiring unit 20 is configured from a communication device to be connected with the server, and the like, and performs data communication with the serve and acquires the image data. Alternatively, the image acquiring unit 20 may be configured from an interface device that inputs an image signal from the endoscope through a cable, and the like.

The input unit 30 is realized by an input device such as a keyboard, a mouse, a touch panel, or various switches, and outputs a received input signal to the control unit 10.

The display unit 40 is realized by a display device such as an LCD or an EL display, and displays various screens including the intraluminal image under control of the control unit 10.

The recording unit 50 is realized by various IC memories such a ROM including an updatable and recordable flash memory and a RAM, a built-in hard disk or hard disk connected with a data communication terminal, or an information recording device such as a CD-ROM, and its reading device. The recording unit 50 stores programs for operating the image processing apparatus 1, and for causing the image processing apparatus 1 to execute various functions, data used during execution of the programs, and the like, in addition to the image data acquired by the image acquiring unit 20. To be specific, the recording unit 50 stores an image processing program 51 for detecting an abnormal portion protruding from a surface of a mucous membrane such as an enlarged fur or polyp from the intraluminal image, various types of information used during execution of the program, and the like.

The calculator 100 is realized by hardware such as a CPU, and applies image processing for the intraluminal image by reading the image processing program 51, and executes the image processing for detecting the abnormal portion protruding from the surface of the mucous membrane such as the enlarged fur or polyp from the intraluminal image.

Next, detailed configurations of the calculator 100 will be described. The calculator 100 includes a contour extracting unit 110 that extracts a plurality of contour pixels from the intraluminal image, an isolated point removing unit 120 that removes an isolated point based on areas of regions of the plurality of contour pixels, a feature data calculator 130 that calculates feature data based on pixel values of the plurality of contour pixels and positional relationship among the plurality of contour pixels, and an abnormal portion detector 140 that detects the abnormal portion based on the feature data.

Among them, the contour extracting unit 110 includes a specific frequency component extracting unit 111 that extracts a region having a specific space frequency component (for example, a region having a space frequency component of a predetermined frequency or more) from the intraluminal image, and an edge extracting unit 112 that extracts an edge from the intraluminal image. The contour extracting unit 110 operates one of the specific frequency component extracting unit 111 and the edge extracting unit 112 to create a specific frequency component image or an edge image, thereby to extract the contour pixel.

The isolated point removing unit 120 connects the contour pixels that configure the same connecting component (that is, the continuing contour pixels), for the contour pixels extracted by the contour extracting unit 110, and removes the contour pixels in a region with an area that is less than a predetermined threshold, of the connected regions, as an isolated point.

The feature data calculator 130 includes a contour end position setting unit 131 that sets an end position to each region (hereinafter, contour region) in which the contour pixels are connected, a circumscribed circle calculator 132 that calculates a center coordinate and a radius of a circumscribed circle of each contour region, a vicinity region setting unit 133 that sets a vicinity region of a position facing the end position on the circumscribed circle, and a pixel value statistic calculator 134 that calculates a statistic of the pixel values of a plurality of pixels in the vicinity region. The feature data calculator 130 outputs the statistic calculated by the pixel value statistic calculator 134 as feature data.

Among them, the contour end position setting unit 131 includes a maximum position calculator 131a that calculates a position of the contour pixel in which at least one of a luminance value and a gradient strength is maximum, from the plurality of contour pixels included in the contour regions, and sets the position of the contour pixel as the end position of the contour region.

Further, the vicinity region setting unit 133 adaptively determines the vicinity region at the position facing the end position, using the radius of the circumscribed circle calculated by the circumscribed circle calculator 132 as a parameter.

The abnormal portion detector 140 determines whether the contour region is an abnormal portion by comparing the feature data (statistic) calculated by the feature data calculator 130 and a predetermined threshold.

Next, the abnormal portion that is an object to be detected by the image processing apparatus 1 will be described with reference to FIGS. 2 and 3. FIG. 2 is a schematic diagram illustrating features of the abnormal portion, and FIG. 3 is a schematic diagram illustrating features of a bubble.

As illustrated in FIG. 2, in the present first embodiment, an enlarged fur (swelling) m1 is detected as the abnormal portion. A swelling m1 has a structure in which an end portion m2 is round and enlarged, and a root portion m3 continues to a mucous membrane surface m4. Therefore, in the intraluminal image, a strong edge appears in the end portion m2, and a region where no edge exists in the root portion m3 that is a facing position to the end portion m2 can be extracted as the swelling m1. Note that, similarly to the swelling m1, an object (for example, a polyp) having a structure protruding from the mucous membrane surface m4 can be extracted according to a similar principle.

Meanwhile, as illustrated in FIG. 3, when seeing a bubble m5 from normal directions of the mucous membrane surface m4, a nearly circular-shaped contour without being disconnected throughout the entire periphery is observed unless noises or dark portions appear. Therefore, strong edges exist in regions m6 and facing regions m6' on the contour of the bubble m5.

Therefore, in the first embodiment, the contour region is extracted from the intraluminal image, and whether a region in a lumen, the region corresponding to the contour region, is the abnormal portion (whether a swelling or a bubble) is determined according to whether the edge exists in the facing position of the contour region.

Hereinafter, a method of processing an image, for detecting the swelling m1 from the intraluminal image, will be described with reference to FIG. 4. FIG. 4 is a flowchart illustrating an operation of the image processing apparatus 1.

First, in step S01, the calculator 100 reads the image data recorded in the recording unit 50, and acquires the intraluminal image that is an object to be processed.

In following step S02, the contour extracting unit 110 selects whether causing the specific frequency component extracting unit 111 to create the specific frequency component image or causing the edge extracting unit 112 to create the edge image, in extracting the contour from the intraluminal image. Here, the specific frequency component refers to a predetermined frequency component selected from among a plurality of space frequency components in the intraluminal image. The contour extracting unit 110 can arbitrarily switch the creation of the specific frequency component image and the creation of the edge image, based on a selection signal input through the input unit 30.

In step S02, when the specific frequency component image is selected, the specific frequency component extracting unit 111 creates the specific frequency component image from the intraluminal image (step S03). Hereinafter, a method of using Fourier transform in this step will be described.

FIG. 5 is a flowchart illustrating processing executed by the specific frequency component extracting unit 111. First, in step S031, the specific frequency component extracting unit 111 converts the intraluminal image into an arbitrary one channel image. As pixel values of pixels that configure the one channel image, for example, R, G, and B channel components, or color ratios G/R, B/G, and the like, of the intraluminal image, are used.

In following step S032, the specific frequency component extracting unit 111 applies two-dimensional Fourier transform to the one channel image, and creates a space frequency component image that is obtained by converting an image space into a frequency space.

In following step S033, the specific frequency component extracting unit 111 depicts a concentric circle with

radiuses $r_1$ and $r_2$ ($r_1 < r_2$), where the center of the space frequency component image becomes the center of the concentric circle.

In step S034, the specific frequency component extracting unit 111 extracts the specific space frequency component by setting pixel values of pixels positioned inside a circle with the radius $r_1$ and pixels positioned outside a circle with the radius $r_2$, to 0. In the present embodiment, the specific frequency component extracting unit 111 extracts high-frequency components having a predetermined frequency or more.

In step S035, the specific frequency component extracting unit 111 converts the frequency space into the image space by applying inverse Fourier transform to the space frequency component image from which the specific space frequency component is extracted. Accordingly, the specific frequency component image including only the specific space frequency component is created. Following that, the processing is returned to the main routine.

Meanwhile, in step S02, when the edge image is selected, the edge extracting unit 112 creates the edge image from the intraluminal image (step S04). To be specific, first, the edge extracting unit 112 converts the intraluminal image into an arbitrary one channel image where the R, G, and B channels or the color ratios G/R, B/G, and the like are the pixel values. Next, the edge extracting unit 112 applies edge extraction processing (Reference: CG-ARTS Association, "Digital Image Processing", pages 114 to 117 (edge extraction)) with a differential filter or a Sobel filter to the one channel image.

In step S05, the contour extracting unit 110 compares the pixel values of the pixels in the specific frequency component image or the edge image, with a predetermined threshold, and sets the pixel values of the pixels, the pixel values being the predetermined threshold or less, to 0, thereby to acquire a contour-extracted image.

In following step S06, the isolated point removing unit 120 removes a pixel wrongly detected as a contour (the pixel is referred to as an isolated point) from the contour-extracted image.

FIG. 6 is a flowchart illustrating processing executed by the isolated point removing unit 120.

In step S061, the isolated point removing unit 120 applies binarization processing with a predetermined threshold to the contour-extracted image. Accordingly, a region with a strong edge having a threshold or more is extracted from the contour-extracted image.

In following step S062, the isolated point removing unit 120 performs region integration by closing (Reference: Corona Publishing Co., Ltd, "Morphology", pages 82 to 90 (expansion to a gray-scale image)) of Morphology processing, for the image to which the binarization processing has been applied, and corrects holes or disconnection due to influence of noises. Note that, as the region integration processing, a region integrating method (Reference: CG-ARTS Association, "Digital Image Processing", page 196) may be applied, instead of the Morphology processing (closing).

In step S063, the isolated point removing unit 120 performs labeling (Reference: CG-ARTS Association, "Digital Image Processing", pages 181 to 182), for the image in which the region integration has been performed, and creates a labeling image that includes regions (label regions) where the pixels that configure the same connecting component are connected. FIG. 7 is a schematic diagram illustrating a creation example of the label ring image. As illustrated in FIG. 7, label regions LB1 to LB5 in a labeling image G1 correspond to regions having a strong edge in the contour-extracted image.

In step S064, the isolated point removing unit 120 calculates areas of the respective label regions LB1 to LB5 in the labeling image G1.

In step S065, the isolated point removing unit 120 sets the pixel values of the regions in the contour-extracted image, the regions corresponding to the label regions with an area having a predetermined threshold or less, to 0. For example, in the case of the labeling image G1 illustrated in FIG. 7, the pixel values of the regions in the contour-extracted image, the regions corresponding to the label regions LB3 to LB5, are set to 0. Accordingly, the isolated points having a strong edge but having a small area are removed from the contour-extracted image.

Following that, the processing is returned to the main routine.

Note that the above-described steps S064 and S065 are executed for improvement of accuracy of following calculation processing, and can be omitted.

In step S07 following step S06, the contour end position setting unit 131 sets end regions to respective contour regions where the contour pixels are connected. FIG. 8 is a flowchart illustrating processing executed by the contour end position setting unit 131. Further, FIG. 9 is a schematic diagram for describing processing of setting the end regions.

In step S071, the maximum position calculator 131a sets the pixel values of the pixels other than the regions corresponding to the label regions of the labeling image created in step S063, to 0, for the contour-extracted image. Note that, in the present first embodiment, the isolated points have already been removed from the contour-extracted image (see step S065). Therefore, a contour-extracted image G2 in which only regions C1 and C2 corresponding to the label regions LB1 and LB2 (see FIG. 7) have pixel values is created by the processing, as illustrated in FIG. 9. These regions C1 and C2 are the contour regions.

In the above-described step S065, the pixel values of the regions in the contour-extracted image other than the regions corresponding to the label regions with an area having a predetermined value or more may be set to 0. In this case, the removal of the isolated points in step S065 and the extraction of the contour regions C1 and C2 in step S071 can be performed at the same time.

In following step S072, the maximum position calculator 131a acquires the pixel values of the pixels in the regions, for each of the contour regions C1 and C2, and acquires a pixel value (hereinafter, referred to as maximum pixel value) of a pixel having the maximum pixel value (luminance value) and position coordinates, from the pixel values.

Here, typically, a plurality of pixels having the maximum pixel value exists in one contour region. Therefore, in step S073, the maximum position calculator 131a integrates adjacent pixels having the maximum pixel value by performing the region integration (Reference: CG-ARTS Association, "Digital Image Processing", page 196).

In step S074, the maximum position calculator 131a sets a region having a maximum area, of the regions integrated in step S073, as the end region of the contour region. Alternatively, the maximum position calculator 131a may set a region having a maximum average value of the pixel values, of the regions integrated in step S073, as the end region. For example, in the case of the contour-extracted image G2, an end region C1' is set to the contour region C1, and an end region C2' is set to the contour region C2. Following that, the processing is returned to the main routine.

In step S08 following step S07, the contour end position setting unit 131 associates the end region set as described above with a label number of the label region corresponding to the contour region that includes the end region.

In step S09, the circumscribed circle calculator 132 calculates center coordinates of the circumscribed circle of the contour region, based on coordinate information of the contour region and the end region. FIG. 10 is a flowchart illustrating processing executed by the circumscribed circle calculator 132. Further, FIG. 11 is a schematic diagram for describing processing of calculating the center coordinates of the circumscribed circle.

First, in step S091, the circumscribed circle calculator 132 applies thinning processing (Reference: CG-ARTS Association, "Digital Image Processing", pages 185 to 186) to the contour regions (for example, the contour regions C1 and C2 in the case of the contour-extracted image G2) in the contour-extracted image from which the isolated points have been removed. FIG. 11 illustrates a region (hereinafter, referred to as thinned region) FL2 from which the contour region C2 illustrated in FIG. 9 has been thinned.

In following step S092, the circumscribed circle calculator 132 performs contour tracking (Reference: CG-ARTS Association, "Digital Image Processing", pages 178 to 179), for the region thinned in step S091, and acquires position coordinates of both end points of the thinned region. For example, for the thinned region FL2, position coordinates ($x_1$, $y_1$) and ($x_2$, $y_2$) of end points $P_{e1}$ and $P_{e2}$ are respectively acquired.

In step S093, the circumscribed circle calculator 132 calculates position coordinates of a gravity center (Reference: CG-ARTS Association, "Digital Image Processing", pages 182 to 183) of the end region of the contour region. For example, in the contour region C2, position coordinates ($x_3$, $y_3$) of a gravity center Pg of the end region C2' are acquired.

In step S094, the circumscribed circle calculator 132 calculates center coordinates of the circumscribed circle from the both end points of the thinned region and the position coordinates of the gravity center. Coordinates ($x_0$, $y_0$) of a center O is provided according to following formulas (1) and (2), using position coordinates ($x_1$, $y_1$) and ($x_2$, $y_2$) of the both end points $P_{e1}$ and $P_{e2}$, and position coordinates ($x_3$, $y_3$) of the gravity center Pg.

$$x_0 = \frac{b_1 c_2 - b_2 c_1}{a_1 b_2 - a_2 b_1} \quad (1)$$

$$y_0 = \frac{c_1 a_2 - c_2 a_1}{a_1 b_2 - a_2 b_1} \quad (2)$$

*Note that,*

$$a_1 = 2(x_2 - x_1)$$

$$b_1 = 2(y_2 - y_1)$$

$$c_1 = x_1^{\ 2} - x_2^{\ 2} + y_1^{\ 2} - y_2^{\ 2}$$

$$a_2 = 2(x_3 - x_1)$$

$$b_2 = 2(y_3 - y_1)$$

$$c_2 = x_1^2 - x_3^2 + y_1^2 - y_3^2$$

The circumscribed circle calculator 132 calculates the center coordinates of the circumscribed circles of the respective contour regions (see FIG. 9), as described above, and stores the center coordinates for each label number.

In step S10 following step S09, radiuses of the circumscribed circles of the respective contour regions are calculated. A radius r of the circumscribed circle is provided by a following formula (3), using the position coordinates ($x_1$, $y_1$) and ($x_2$, $y_2$) of the both end points $P_{e1}$ and $P_{e2}$ and the position coordinates ($x_3$, $y_3$) of the gravity center $P_g$.

$$r = \frac{1}{2}\frac{\sqrt{\{(x_1 - x_2)^2 + (y_1 - y_2)^2\}\{(x_2 - x_3)^2 + (y_2 - y_3)^2\}\{(x_3 - x_1)^2 + (y_3 - y_1)^2\}}}{|x_1(y_2 - y_3) + x_2(y_3 - y_1) + x_3(y_1 - y_2)|} \quad (3)$$

The circumscribed circle calculator 132 calculates the radius of the circumscribed circles of the respective contour regions (see FIG. 9), as described above, and stores the radius r for each label number.

In following step S11, the vicinity region setting unit 133 acquires vicinity regions in positions facing the contour regions in the circumscribed circles, for the respective contour regions, for each label number. FIG. 12 is a flowchart illustrating processing executed by the vicinity region setting unit 133. Further, FIGS. 13 and 14 are schematic diagrams for describing processing of acquiring the vicinity regions.

In step S111, the vicinity region setting unit 133 calculates coordinates of a contour facing position pixel, from the position of the gravity center of the end region. To be specific, as illustrated in FIG. 13, the vicinity region setting unit 133 connects the gravity center $P_g$ of the end region and the center O of the circumscribed circle CS, and employs an intersection point pixel $P_c$ of a line extending from the center O by the radius r and the circumscribed circle CS, as the contour facing position pixel.

In step S112, the vicinity region setting unit 133 sets a vicinity region having the contour facing position pixel $P_c$ as a center. This is because it is not favorable in terms of accuracy to determine existence/non-existence of the edge in the facing position of the contour region only with one point of the contour facing position pixel $P_c$.

Therefore, the vicinity region setting unit 133 acquires a predetermined region having the contour facing position pixel $P_c$ as the center, as the vicinity region. To be specific, as illustrated in FIG. 14, the vicinity region setting unit 133 employs an arc-shaped region with a width $\Delta r$, excluding a fan shape with a center angle $\theta$ and a radius $r_a$ ($r_a < r$) from a fan shape of a center angle 9 and a radius $r_b$ ($r_b > r$) in which the contour facing position pixel $P_c$ is the center, as a vicinity region N.

Note that the vicinity region is not limited to the above-described arc-shaped region, and simply, for example, a rectangle region, a circle region, or an ellipse region, having the contour facing position pixel $P_c$ as the center, may be employed as the vicinity region. In this case, the length of one side of the rectangle region, the diameter of the circle region, or the length of the axis of the ellipse region may be adaptively determined according to the radius r of the circumscribed circle CS such that the vicinity region can become a shape as similar as possible to the circumscribed circle CS.

In step S12, the pixel value statistic calculator 134 calculates an average value, as a statistic of the pixel values in the vicinity region set for each label, in the contour-extracted image. Note that the pixel value statistic calculator 134 may calculate a maximum value and a most-frequent value, as the statistics, in addition to the average value.

In step S13, the abnormal portion detector 140 determines whether the contour region is the abnormal portion for each label, by comparing the average value calculated in step S12 and a predetermined threshold. To be specific, when the average value is larger than the threshold, that is, when a high-frequency component or a strong edge exists in the vicinity region facing the contour region, the abnormal portion detector 140 determines that the contour region is not the abnormal portion (that is, is a bubble region). On the other hand, when the average value is the threshold or less, that is, when the high-frequency component or the strong edge does not exist in the vicinity region facing the contour region, the abnormal portion detector 140 determines that the contour region is the abnormal portion such as a swelling.

In step S14, the calculator 100 outputs a detection result of the abnormal portion to record the detection result in the recording unit 50, and displays the detection result in the display unit 40.

As described above, according to the first embodiment, the contour region is extracted from the intraluminal image, and whether the contour region is the abnormal portion is determined based on the pixel values (luminance values) of the pixels in the contour region and the positional relationship. Therefore, the abnormal portion protruding from the surface of the mucous membrane and the bubble are clearly distinguished, and the abnormal portion can be accurately detected.

(Modification 1-1)

Next, a modification 1-1 of the first embodiment will be described.

In the first embodiment, the specific frequency component image has been created using Fourier transform and inverse Fourier transform. However, an image made of a specific frequency component can be created by difference of Gaussian (DOG). In the present modification 1-1, processing of creating a specific frequency component image by the DOG will be described. FIG. 15 is a flowchart illustrating processing of creating a specific frequency component image. Note that step S031' illustrated in FIG. 15 corresponds to step S031 illustrated in FIG. 5.

In step S032' following step S031', a specific frequency component extracting unit 111 calculates a smoothed image $L_i$ by performing a convolution operation of an arbitrary one channel image created from an intraluminal image, and a Gaussian function of a scale $\sigma_0 = \sigma_0$. Here, the reference sign i is a parameter indicating the number of times of calculations, and i = 1 is set as an initial value.

In following step S033', the specific frequency component extracting unit 111 calculates a smoothed image $L_{i+1}$ by performing the convolution operation of the smoothed image $L_i$ and the Gaussian function of the scale $\sigma = k^i\sigma_0$. Here, the reference number k indicates an increase rate of the Gaussian function.

In step S034', the specific frequency component extracting unit 111 determines whether further repeating the convolution operation. When repeating the convolution operation (Yes in step S034'), the specific frequency component extracting unit 111 increments the parameter i (i = i + 1, in step S035'). Following that the processing is moved onto step S033'.

Meanwhile, when not repeating the convolution operation (No in step S034'), the specific frequency component extracting unit 111 acquires a difference image between arbitrary two smoothed images $L_{i=n}$, $L_{i=m}$ (n and m are natural numbers) (step S036'). Following that, the processing is returned to the main routine. This difference image can be used as the specific frequency component image in step S05.

(Modification 1-2)

In the first embodiment, the region of the pixel having the maximum pixel value in the contour region is employed as the end region (see step S07). However, a region of a pixel having a maximum gradient of a pixel value (luminance value) in a contour region may be employed as an end region. In this case, a contour end position setting unit 131 acquires the gradient of the pixel having the maximum inline and position coordinates, for each contour region. In this case, when a plurality of pixels having the maximum gradient is acquired, region division is performed by integration of adjacent pixels (Reference: CG-ARTS Association, "Digital Image Processing", page 196), and a region having a maximum average value of the gradient may just be set as the end region.

(Second Embodiment)

Next, a second embodiment of the present invention will be described.

FIG. 16 is a block diagram illustrating a configuration of an image processing apparatus according to the second embodiment. As illustrated in FIG. 16, an image processing apparatus 2 according to the second embodiment includes a calculator 200 including a contour extracting unit 210, a feature data calculator 220, and an abnormal portion detector 230, instead of the calculator 100 illustrated in FIG. 1. Note that configurations and operations of respective units of the image processing apparatus 2 other than the calculator 200 are similar to the first embodiment.

The contour extracting unit 210 includes a circular-shaped contour extracting unit 211 that extracts a plurality of contour pixels from an intraluminal image, and estimates a circular-shaped region with a circumference, at least a part of which is formed of these contour pixels, based on the plurality of contour pixels. Hereinafter, the circular-shaped region estimated by the contour extracting unit 210 is referred to as circular contour.

The feature data calculator 220 includes a maximum-value minimum-value position calculator 221 that calculates position coordinate of a pixel having a maximum pixel value (hereinafter, referred to as maximum pixel value) and of a pixel having a minimum pixel value (hereinafter, referred to as minimum pixel value), of pixels on the circular contour, and an angle calculator 222 that calculates an angle made by a line segment that connects the pixel having the maximum pixel value and the pixel having the minimum pixel value on the circular contour, and a normal line in a position of the pixel having the maximum pixel value, and outputs the angle calculated by the angle calculator 222 as feature data based on the pixel values of the plurality of contour pixels and the positional relationship.

The abnormal portion detector 230 determines whether the circular contour is an abnormal portion, based on the angle output as the feature data.

Next, an operation of the image processing apparatus 2 will be described.

FIG. 17 is a diagram for describing a go-around profile of the abnormal portion in the circular contour. In the present second embodiment, the circular contour is estimated by applying a circular shape to the contour pixel extracted from the intraluminal image, and pixel value change on the circular contour is acquired. Here, as illustrated in FIG. 17, in an image that captures a swelling m11, a strong edge appears in an end portion m12. However, no strong edge appears in a facing position, that is, in a root portion m14 continuing with a mucous membrane surface m13. Therefore, when observing the pixel value change (hereinafter, referred to as go-around profile) along a circular contour m15 corresponding to the swelling m11, a pixel $P_{min}$ having a minimum pixel value $V_{min}$ exists in nearly the facing position of a pixel $P_{max}$ having a maximum pixel value $V_{max}$. Note that, in the graph on the left side of FIG. 17, the horizontal axis represents the position coordinates of when a locus on the circular contour m15 is converted into a straight line.

Meanwhile, in an image that captures a bubble, an edge continuing in a nearly circular shape appears unless there is influence of noises or dark portions. Therefore, in a go-around profile of the circular contour corresponding to the bubble, variation of the pixel values is small, including the facing position, and regular positional relationship between the pixel $P_{max}$ and the pixel $P_{min}$ like the case of the swelling m11 is not observed.

Therefore, in the second embodiment, the pixel $P_{min}$ having the minimum pixel value $V_{min}$ and the pixel $P_{max}$ having the maximum pixel value $V_{max}$ from the go-around profile of the circular contour m15 estimated in the intraluminal image, and whether a region in a lumen corresponding to the circular contour m15 is an abnormal portion (a swelling or a bubble) is determined based on the positional relationship between the pixels $P_{max}$ and $P_{min}$.

FIG. 18 is a flowchart illustrating an operation of the image processing apparatus 2. Note that step S21 illustrated in FIG. 18 corresponds to step S01 of FIG. 4.

In step S22 following step S21, the circular-shaped contour extracting unit 211 extracts the contour pixels from the intraluminal image, and estimates the circular-shaped region with a circumference, at least a part of which is formed of the contour pixels, based on the contour pixels. FIG. 19 is a flowchart illustrating processing executed by the circular-shaped contour extracting unit 211.

First, in step S221, the circular-shaped contour extracting unit 211 converts the intraluminal image into an arbitrary one channel image. As the pixel values of the pixels in the one channel image, R, G, and B channels or color ratios G/R, B/G, and the like in the intraluminal image are used.

In following step S222, the circular-shaped contour extracting unit 211 calculates gradient strengths of the pixel values of the pixels by applying edge extraction processing (Reference: CG-ARTS Association, "Digital Image Processing", pages 114 to 121) with a Laplacian filter or a Sobel filter to the one channel image. Hereinafter, an image having the calculated gradient strengths as the pixel values is referred to as gradient strength image.

In step S223, the circular-shaped contour extracting unit 211 applies binarization processing to the gradient strength image calculated in step S222, and extracts a pixel having a stronger gradient strength (stronger edge pixel) than a predetermined threshold, thereby to create an edge image.

In step S224, the circular-shaped contour extracting unit 211 estimates the circular-shaped region along the strong edge pixel (that is, the contour) by applying circle-applying processing to the edge image. As the circle-applying processing, known calculation processing such as Hough conversion (Reference: CG-ARTS Association, "Digital Image Processing", pages 211 to 214) can be used, for example. Here, the Hough conversion is processing of voting for initial candidate points to a parameter space made of a radius of a circle and center coordinates of the circle, calculating an evaluation value for detecting the circular shape based on the frequency of voting in the parameter space, and determining the circular shape based on the evaluation value. Alternatively, processing of extracting an edge as a closed curve, such as Sneak (Snakes, Reference: CG-ARTS Association, "Digital Image Processing", pages 197 to 198) may be executed instead of the circle-applying processing.

The circular-shaped region estimated as described above is output as the circular contour. Following that, the processing is returned to the main routine.

In step S23 following step S22, the contour extracting unit 210 creates a circular contour extraction labeled image to which a label is provided to each circular contour estimated in step S22. To be specific, the contour extracting unit 210 sets the pixel values in the circular contour to 1, and sets pixel values in other regions to 0, thereby to create a binarized image. Then, the contour extracting unit 210 performs labeling to the binarized image.

In step S24, the maximum-value minimum-value position calculator 221 obtains the position coordinates of the pixel having the maximum pixel value and the pixel having the minimum pixel value in the circular contour for each label. FIG. 20 is a flowchart illustrating processing executed by the maximum-value minimum-value position calculator 221.

In step S241, the maximum-value minimum-value position calculator 221 performs raster scan in the circular contour extraction labeled image, and determines a starting position of the go-around profile on the circular contour.

In following step S242, the maximum-value minimum-value position calculator 221 scans the circular contour extraction labeled image along the circular contour, and stores the pixel values of the pixels corresponding to the one channel image and the position coordinates. Accordingly, the go-around profile can be obtained. To perform scanning along the circular contour, for example, contour tracking (Reference: CG-ARTS Association, "Digital Image Processing", page 178) may be favorably used.

In step S243, the maximum-value minimum-value position calculator 221 extracts the maximum pixel value and the minimum pixel values from the go-around profile, and obtains the position coordinates of the pixel having the maximum pixel value and the pixel having the minimum pixel value. Following that, the processing is returned to the main routine.

In step S25 following step S24, the angle calculator 222 calculates feature data that indicates the positional relationship between the pixel having the maximum pixel value and the pixel having the minimum pixel value. To be specific, as illustrated in FIG. 21, the angle calculator 222 calculates an angle $\alpha$ made by a line segment m16 connecting the pixel $P_{max}$ having the maximum pixel value $V_{max}$ and the pixel $P_{min}$ having the minimum pixel value $V_{min}$, and a normal line m17 in the pixel $P_{max}$, on the circular contour m15, as the feature data. The angle calculator 222 calculates and stores such an angle $\alpha$ for each label.

In step S26, the abnormal portion detector 230 determines whether the circular contour is the abnormal portion for each label by comparing the angle $\alpha$ calculated as the feature data and a pedetermined threshold. To be specific, when the angle $\alpha$ is larger than the predetermined threshold, that is, when the positional relationship between the pixel $P_{max}$ and the pixel $P_{min}$ deviates from the facing position on the circular contour m15, the abnormal portion detector 230 determines that the circular contour is not the abnormal portion (that is, is a bubble). On the other hand, when the angle $\alpha$ is the threshold or less, that is, when the positional relationship between the pixel $P_{max}$ and the pixel $P_{min}$ is close to the facing position on the circular contour m15, the abnormal portion detector 230 determines that the circular contour is the abnormal portion such as a swelling.

In step S27, the calculator 200 outputs a detection result of the abnormal portion and records the detection result in a recording unit 50, and displays the detection result in a display unit 40.

As described above, according to the second embodiment, the circular contour is estimated from the contour pixels extracted from the intraluminal image, and whether the circular contour is the abnormal portion is determined based on the positional relationship between the pixel having the maximum pixel value and the pixel having the minimum pixel value in the circular contour. Therefore, the abnormal portion protruding from a surface of a mucous membrane and a bubble are clearly distinguished, and the abnormal portion can be accurately detected.

(Modification 2-1)

In the second embodiment, the gradient strengths in the one channel image created from the intraluminal image are calculated, and the contour pixels are extracted based on the gradient strengths of the pixels. However, a specific frequency component image (a high-frequency component image in this modification) may be created from one channel image, and contour pixels may be extracted from the specific frequency component image. Note that processing of creating the specific frequency component image is similar to the first embodiment.

(Third Embodiment)

Next, a third embodiment of the present invention will be described.

FIG. 22 is a block diagram illustrating a configuration of an image processing apparatus according to the third embodiment. As illustrated in FIG. 22, an image processing apparatus 3 according to the third embodiment includes a calculator 300 including a contour extracting unit 210, a feature data calculator 310, and an abnormal portion detector 320, instead of the calculator 200 illustrated in FIG. 16. Note that configurations and operations of respective units of the image processing apparatus 3 other than the calculator 300 are similar to the first embodiment. Further, a configuration and an operation of the contour extracting unit 210 in the calculator 300 is similar to the second embodiment.

The feature data calculator 310 includes a facing position pixel correlation value calculator 312 that extracts a pixel on a circular contour output from the contour extracting unit 210, and a pixel (hereinafter, referred to as facing position pixel) in a facing position relationship with the pixel, and calculates a correlation value of pixel values between these facing pixels, and outputs a statistic or distribution of the correlation value as feature data.

The abnormal portion detector 320 determines whether a circular contour is an abnormal portion based on the statistic or the distribution of the correlation value of the pixel values between the facing pixels on the circular contour.

Next, an operation of the image processing apparatus 3 will be described. FIG. 23 is a schematic diagram for describing features of the pixel values on the circular contour in a swelling as the abnormal portion. Further, FIG. 24 is a schematic diagram for describing features of the pixel values on the circular contour in a bubble.

In the third embodiment, the circular contour is estimated by applying a circular shape to contour pixels extracted from an intraluminal image, and the correlation value of the pixel values between the facing pixels on the circular contour. Here, as illustrated in FIG. 23, in an image that captures a swelling m21, a strong edge appears in an end portion m22. However, no strong edge appears in a facing position of the end portion m22, that is, in a root portion m24 continuing to a mucous membrane surface m23. Therefore, in a direction (see the both arrow OP1) connecting the end portion m22 and the root portion m24 of the swelling m21, a difference in the pixel values between the facing pixels on a circular contour m25 becomes large. Meanwhile, in sides of the swelling m21, an edge is basically observed regardless of directions. Therefore, in a direction (see the both arrows OP2 and OP3) connecting the sides of the swelling m21, the difference in the pixel values between the facing pixels on the circular contour m25 becomes small. Therefore, when the difference in the pixel values between the facing pixels on the circular contour m25 is acquired throughout a round, combinations of the pixels having a large difference in the pixel values are mixed, and variation of the difference in the pixel values becomes large.

Meanwhile, as illustrated in FIG. 24, in an image that captures a bubble m26, an edge continuing in a nearly circular shape appears unless there is influence of noises and dark portions. Therefore, the strength of the edge is similar in any position on the circular contour m25 corresponding to the bubble m26. Therefore, the difference in the pixel values of the facing pixels on the circular contour m25 basically becomes a small value regardless of directions (the both arrows OP4 to OP6), and the variation of the difference in the pixel values becomes small.

Therefore, in the third embodiment, the correlation value (difference) in the pixel values between the facing pixels on the circular contour m25 estimated in the intraluminal image is acquired throughout a round, and whether a region in a lumen corresponding to the circular contour m25 is an abnormal portion (a swelling or a bubble) is determined based on a statistic or distribution of the correlation value.

FIG. 25 is a flowchart illustrating an operation of the image processing apparatus 3. Note that steps S31 to S33 illustrated in FIG. 25 correspond to steps S21 to S23 in FIG. 18. Note that, in step S32, the contour pixels may be extracted from a specific frequency component image, similarly to the modification 2-1.

In step S34 following step S33, the facing position pixel correlation value calculator 312 calculates the correlation value of the pixel values between the mutually facing pixels on the circular contour of each label. FIG. 26 is a flowchart illustrating processing executed by the facing position pixel correlation value calculator 312. Further, FIG. 27 is a schematic diagram for describing processing of calculating the correlation value.

First, in step S341, the facing position pixel correlation value calculator 312 performs raster scan in a circular contour extraction labeled image, and determines a pixel having a value first, as a starting point of the correlation value calculation. In FIG. 27, a pixel $P_1$ is the starting point.

Following that, the facing position pixel correlation value calculator 312 executes processing of a loop A throughout a half round of the circular contour m25.

In step S342, the facing position pixel correlation value calculator 312 acquires the pixel value of a target pixel on the circular contour m25 and the pixel value of a facing position pixel of the target pixel, and stores the pixel values as pair pixel values. Note that the pixel $P_1$ is set to the target pixel in the first time.

In step S343, the facing position pixel correlation value calculator 312 moves the position of the target pixel along the circular contour m25 by a predetermined amount by contour tracking (Reference: CG-ARTS Association, "Digital Image Processing", page 178).

By repetition of these steps S342 and S343, the pair pixel values of target pixels $P_1$, $P_2$, $P_3$, ... and facing position pixels $P_{c1}$, $P_{c2}$, $P_{c3}$, ... are sequentially stored. Such processing is continued until the target pixels $P_1$, $P_2$, $P_3$, ... cover the half round of the circular contour m25.

In step S344, the facing position pixel correlation value calculator 312 calculates the correlation values in the respective pair pixel values. To be specific, an absolute value or a square value of the difference in the pixel values between the mutually facing pixels is calculated.

Following that, the processing is returned to the main routine.

In step S35 following step S34, the feature data calculator 310 calculates the statistic of the correlation values calculated for the pair pixel values in step S34. To be specific, a maximum value of the correlation values or a value of dispersion of the correlation values is calculated.

In step S36, the abnormal portion detector 320 determines whether the circular contour is the abnormal portion for each label by comparing the statistic calculated as the feature data and a threshold. To be specific, the abnormal portion detector 320 determines that the circular contour m25 is the abnormal portion when the statistic is the predetermined threshold or more. On the other hand, the abnormal portion detector 320 determines that the circular contour m25 is not the abnormal portion (that is, is the bubble) when the statistic is smaller than the predetermined threshold.

In step S37, the calculator 300 outputs a detection result of the abnormal portion and records the detection result in a recording unit 50, and displays the detection result in a display unit 40.

As described above, according to the third embodiment, the circular contour is estimated from the contour-extracted from the intraluminal image, and whether the circular contour is an abnormal portion is determined based on the correlation value of the pixel value between the pixels facing on the circular contour. Therefore, the abnormal portion protruding from the surface of the mucous membrane and a bubble are clearly distinguished, and the abnormal portion can be accurately detected.

(Modification 3-1)

In the third embodiment, the abnormal portion has been determined based on the statistic of the correlation value between the pair pixel values. However, the abnormal portion may be determined based on distribution of the pair pixel values. In the modification 3-1, processing of determining an abnormal portion based on distribution of pair pixel values will be described.

In this case, after pair pixel values are acquired by processing in a loop A of FIG. 26, a feature data calculator 310 creates distribution obtained by projecting pair pixel values having a pixel value of a target pixel (first point pixel value) and a pixel value of a facing position pixel (second point pixel value) as components into a multidimensional space, as illustrated in FIG. 28. An abnormal portion detector 320 performs processing of determining an abnormal portion, for the distribution of the pair pixel values, by a partial space method (Reference: CG-ARTS Association, "Digital Image Processing", pages 229 to 230) or the like. To be specific, in FIG. 28, when the pair pixel values are distributed in regions A1 and A2 where a difference in the first point pixel value and the second point pixel value is large, a circular contour is determined to be the abnormal portion.

The image processing apparatuses according to the above-described first to third embodiments and its modifications can be realized by execution of an image processing program recorded in a recording device by a computer system such as a personal computer or a work station. Further, such a computer system may be used by being connected with a device such as another computer system or a server, through a local region network, a broadband region network (LAN/WAN), or a public line such as the Internet. In this case, the image processing apparatuses according to the first to third embodiments and its modifications may acquire image data of an intraluminal image through these networks, may output an image processing result to various types of output devices (a viewer or a printer) connected through these networks, or may store the image processing result in storage devices (a recording device and its reading device) connected to these networks.

Note that the present invention is not limited to the first to third embodiments and its modifications, and various inventions can be formed by appropriate combination of a plurality of configuration elements disclosed in the embodiments and modifications. For example, the various inventions may be formed by exclusion of some of all the configuration elements described in the embodiments and modifications, or may be formed by appropriate combination of the configuration elements described in the different embodiments and modifications.

Reference Signs List

| | |
|---|---|
| 1 to 3 | IMAGE PROCESSING APPARATUS |
| 10 | CONTROL UNIT |
| 20 | IMAGE ACQUIRING UNIT |
| 30 | INPUT UNIT |
| 40 | DISPLAY UNIT |
| 50 | RECORDING UNIT |
| 51 | IMAGE PROCESSING PROGRAM |
| 100, 200, and 300 | CALCULATION UNIT |
| 110 and 210 | CONTOUR EXTRACTING UNIT |
| 111 | SPECIFIC FREQUENCY COMPONENT EXTRACTING UNIT |
| 112 | EDGE EXTRACTING UNIT |
| 120 | ISOLATED POINT REMOVING UNIT |
| 130, 220, and 310 | FEATURE DATA CALCULATOR |
| 131a | MAXIMUM POSITION CALCULATOR |
| 131 | CONTOUR END POSITION SETTING UNIT |
| 132 | CIRCUMSCRIBED CIRCLE CALCULATOR |
| 133 | VICINITY REGION SETTING UNIT |
| 134 | PIXEL VALUE STATISTIC CALCULATOR |
| 140, 230, and 320 | ABNORMAL PORTION DETECTOR |
| 211 | CIRCULAR-SHAPED CONTOUR EXTRACTING UNIT |
| 221 | MAXIMUM-VALUE MINIMUM-VALUE POSITION CALCULATOR |
| 222 | ANGLE CALCULATOR |
| 312 | FACING POSITION PIXEL CORRELATION VALUE CALCULATOR |

## Claims

1. An image processing apparatus comprising:

   a contour extracting unit configured to extract a plurality of contour pixels from an image acquired by capturing an inside of a lumen of a living body;
   a feature data calculating unit configured to calculate feature data based on pixel values of the plurality of contour pixels and positional relationship among the plurality of contour pixels; and
   an abnormal portion detecting unit configured to detect an abnormal portion in the lumen based on the feature data.

2. The image processing apparatus according to claim 1, wherein the contour extracting unit includes a circular-shaped contour extracting unit that is configured to extract a plurality of contour pixels from the image and estimate a circular-shaped region with a circumference, at least a part of the circumference being formed of the plurality of contour pixels, and
the feature data calculating unit includes a maximum-value minimum-value position calculating unit that is configured to calculate position coordinates on the image, of a pixel having a maximum pixel value and a pixel having a minimum pixel value, of the pixels on the contour forming the circular shape.

3. The image processing apparatus according to claim 2, wherein the feature data calculating unit includes an angle calculating unit that is configured to calculate an angle made by a line segment connecting the pixel having a maximum pixel value and the pixel having a minimum pixel value and a normal line in a position of the pixel having a maximum pixel value, and
the abnormal portion detecting unit determines that the region of the plurality of contour pixels is the abnormal portion when the angle is a predetermined value or less.

4. The image processing apparatus according to claim 1, wherein the contour extracting unit includes a circular-shaped contour extracting unit that is configured to extract a plurality of contour pixels from the image and estimate a contour forming a circular shape based on the plurality of contour pixels,
the feature data calculating unit includes a facing position pixel correlation value calculating unit that is configured to extract mutually facing pixels on the contour forming a circular shape and calculate a correlation value of pixel values between the mutually facing pixels, and
the abnormal portion detecting unit detects whether a region of the plurality of contour pixels is the abnormal portion based on the correlation value.

5. The image processing apparatus according to claim 4, wherein the correlation value is an absolute value or a square value of a difference of the pixel values between the mutually facing pixels, and
the abnormal portion detecting unit determines that the region of the plurality of contour pixels is the abnormal portion when a statistic of the correlation values calculated throughout an entire periphery of the contour forming a circular shape is a predetermined threshold or more.

6. The image processing apparatus according to claim 4, wherein the correlation values are distribution in a multidimensional space in which the respective pixel values of the mutually facing pixels are components, and
the abnormal portion detecting unit determines that the region of the plurality of contour pixels is the abnormal portion when a combination of the pixel values of the mutually facing pixels is distributed in a predetermined region in the multidimensional space based on the distribution.

7. The image processing apparatus according to claim 1, further comprising:

   an isolated point removing unit configured to remove an isolated point based on an area of a region where the contour pixels are connected.

8. The image processing apparatus according to claim 1, wherein the feature data calculating unit includes:

   a contour end position setting unit that is configured to set an end position in a contour region that is a region where the contour pixels are connected;
   a circumscribed circle calculating unit that is configured to calculate a circumscribed circle of the contour region;
   a vicinity region setting unit that is configured to set a vicinity region in a position facing the end position on the circumscribed circle; and
a pixel value statistic calculating unit that is configured to calculate a statistic of pixel values of a plurality of pixels in the vicinity region.

**9.** The image processing apparatus according to claim 8, wherein
the contour end position setting unit includes:

a maximum value pixel position calculating unit that is configured to calculate a position of a contour pixel in which at least one of a luminance value and a gradient strength is maximum, from the plurality of contour pixels included in the contour region, and

the contour end position setting unit sets the position of the contour pixel having the maximum luminance value or the maximum gradient strength as the end position.

**10.** The image processing apparatus according to claim 9, wherein the abnormal portion detecting unit calculates a correlation between the statistic of the pixel values of the plurality of pixels in the vicinity region, and a statistic of the pixel value of the contour pixel in the end position, and determines that the region of the plurality of contour pixels is the abnormal portion when the correlation is low.

**11.** A method of processing an image, the method comprising:

a contour extracting step of extracting a plurality of contour pixels from an image obtained by capturing an inside of a lumen of a living body;
a feature data calculating step of calculating feature data based on pixel values of the plurality of contour pixels and positional relationship among the plurality of contour pixels; and
an abnormal portion detecting step of detecting an abnormal portion based on the feature data.

**12.** An image processing program for causing a computer to execute:

a contour extracting step of extracting a plurality of contour pixels from an image obtained by capturing an inside of a lumen of a living body;
a feature data calculating step of calculating feature data based on pixel values of the plurality of contour pixels and positional relationship among the plurality of contour pixels; and
an abnormal portion detecting step of detecting an abnormal portion based on the feature data.

FIG.1

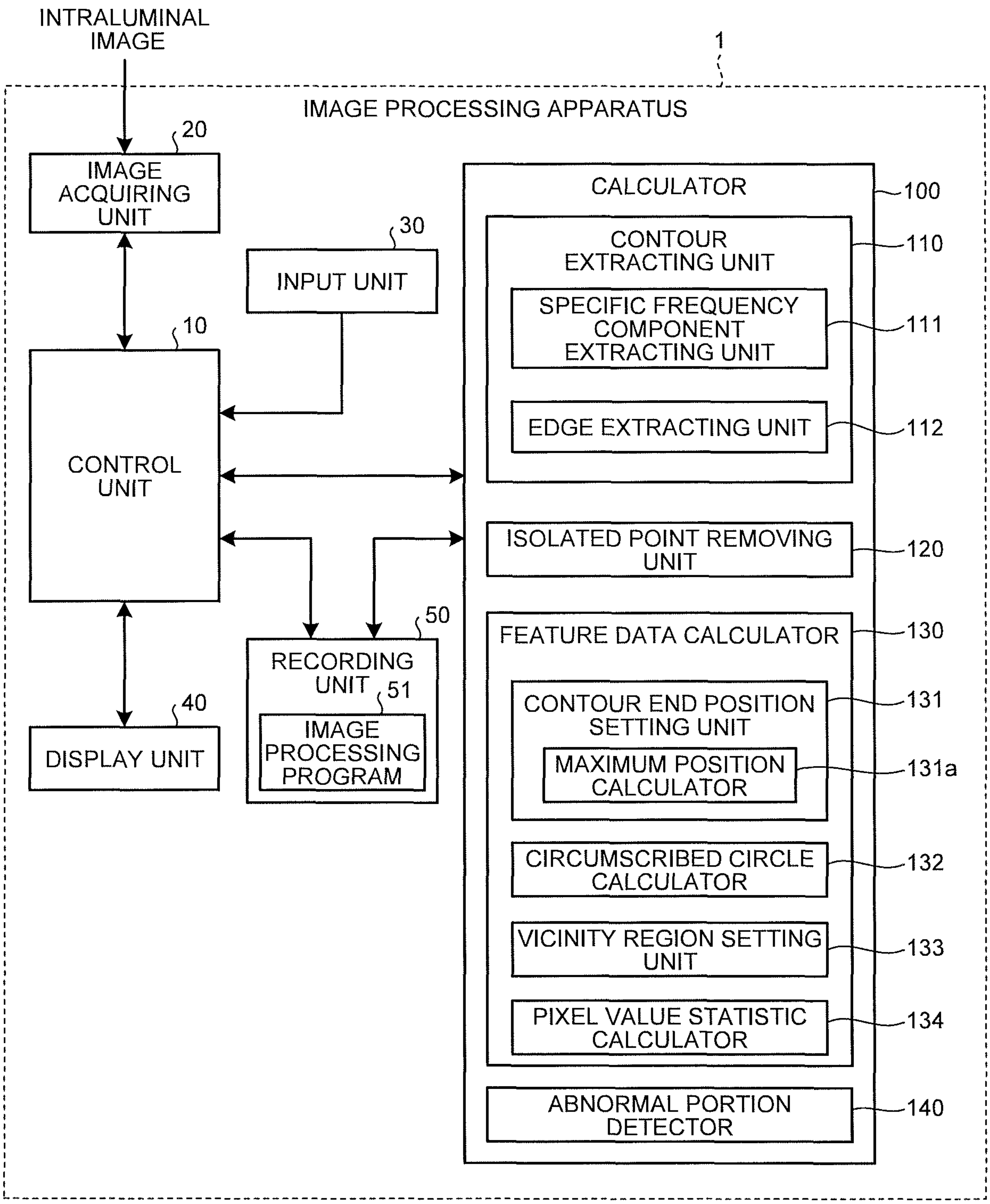
INTRALUMINAL IMAGE
1
IMAGE PROCESSING APPARATUS
20
IMAGE ACQUIRING UNIT
30
INPUT UNIT
10
CONTROL UNIT
40
DISPLAY UNIT
50
RECORDING UNIT
51
IMAGE PROCESSING PROGRAM
CALCULATOR
100
CONTOUR EXTRACTING UNIT
110
SPECIFIC FREQUENCY COMPONENT EXTRACTING UNIT
111
EDGE EXTRACTING UNIT
112
ISOLATED POINT REMOVING UNIT
120
FEATURE DATA CALCULATOR
130
CONTOUR END POSITION SETTING UNIT
131
MAXIMUM POSITION CALCULATOR
131a
CIRCUMSCRIBED CIRCLE CALCULATOR
132
VICINITY REGION SETTING UNIT
133
PIXEL VALUE STATISTIC CALCULATOR
134
ABNORMAL PORTION DETECTOR
140

FIG.2

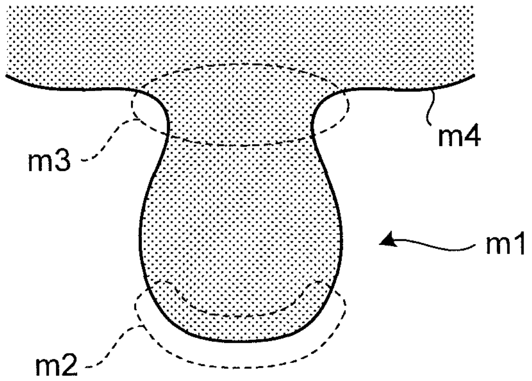


FIG.3

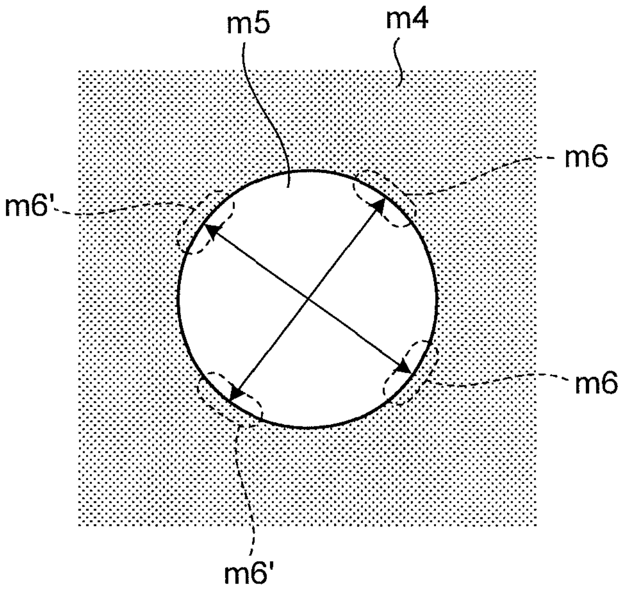

FIG.4

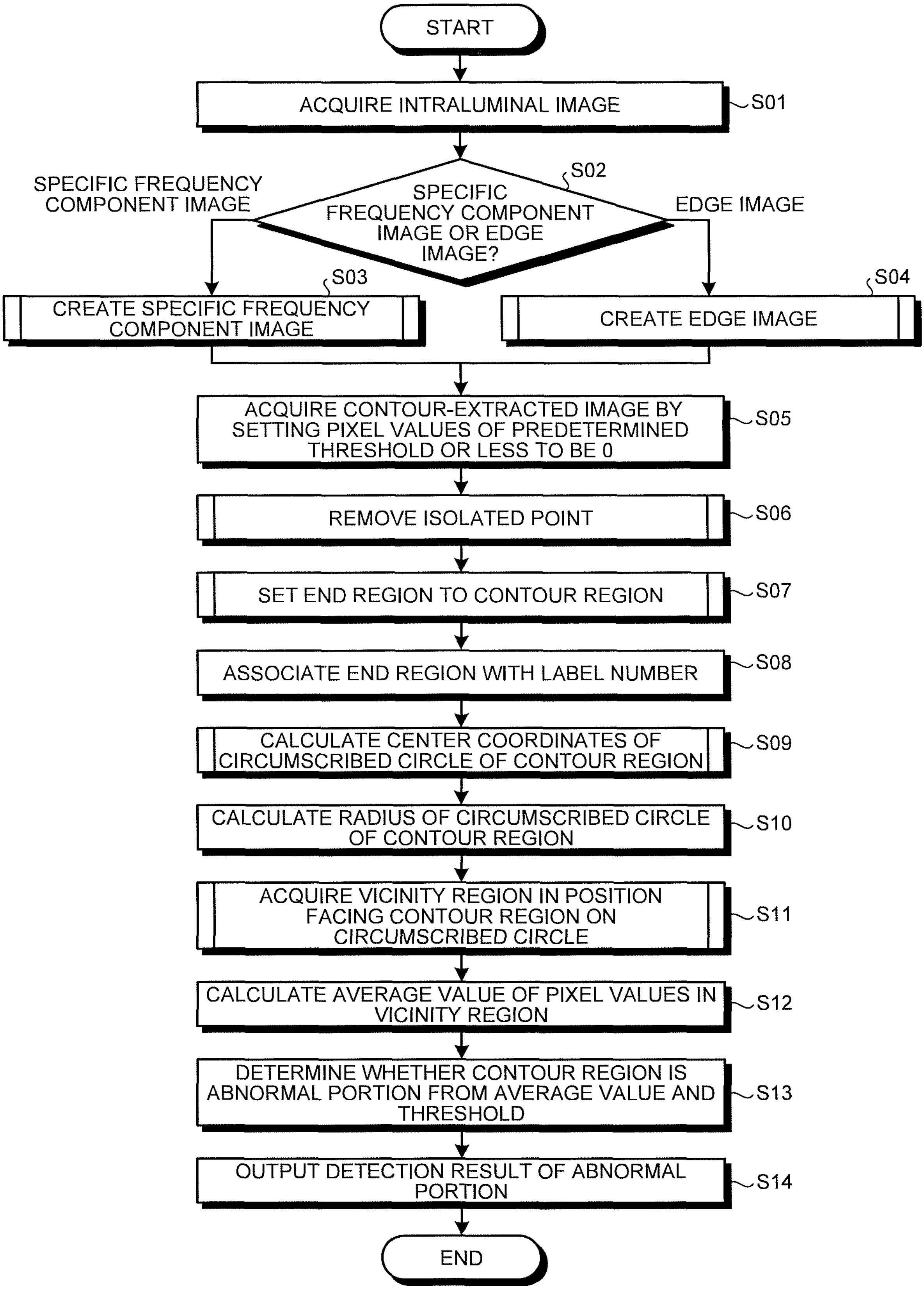
START
ACQUIRE INTRALUMINAL IMAGE
S01
SPECIFIC FREQUENCY COMPONENT IMAGE OR EDGE IMAGE?
S02
SPECIFIC FREQUENCY COMPONENT IMAGE
EDGE IMAGE
CREATE SPECIFIC FREQUENCY COMPONENT IMAGE
S03
CREATE EDGE IMAGE
S04
ACQUIRE CONTOUR-EXTRACTED IMAGE BY SETTING PIXEL VALUES OF PREDETERMINED THRESHOLD OR LESS TO BE 0
S05
REMOVE ISOLATED POINT
S06
SET END REGION TO CONTOUR REGION
S07
ASSOCIATE END REGION WITH LABEL NUMBER
S08
CALCULATE CENTER COORDINATES OF CIRCUMSCRIBED CIRCLE OF CONTOUR REGION
S09
CALCULATE RADIUS OF CIRCUMSCRIBED CIRCLE OF CONTOUR REGION
S10
ACQUIRE VICINITY REGION IN POSITION FACING CONTOUR REGION ON CIRCUMSCRIBED CIRCLE
S11
CALCULATE AVERAGE VALUE OF PIXEL VALUES IN VICINITY REGION
S12
DETERMINE WHETHER CONTOUR REGION IS ABNORMAL PORTION FROM AVERAGE VALUE AND THRESHOLD
S13
OUTPUT DETECTION RESULT OF ABNORMAL PORTION
S14
END

FIG.5

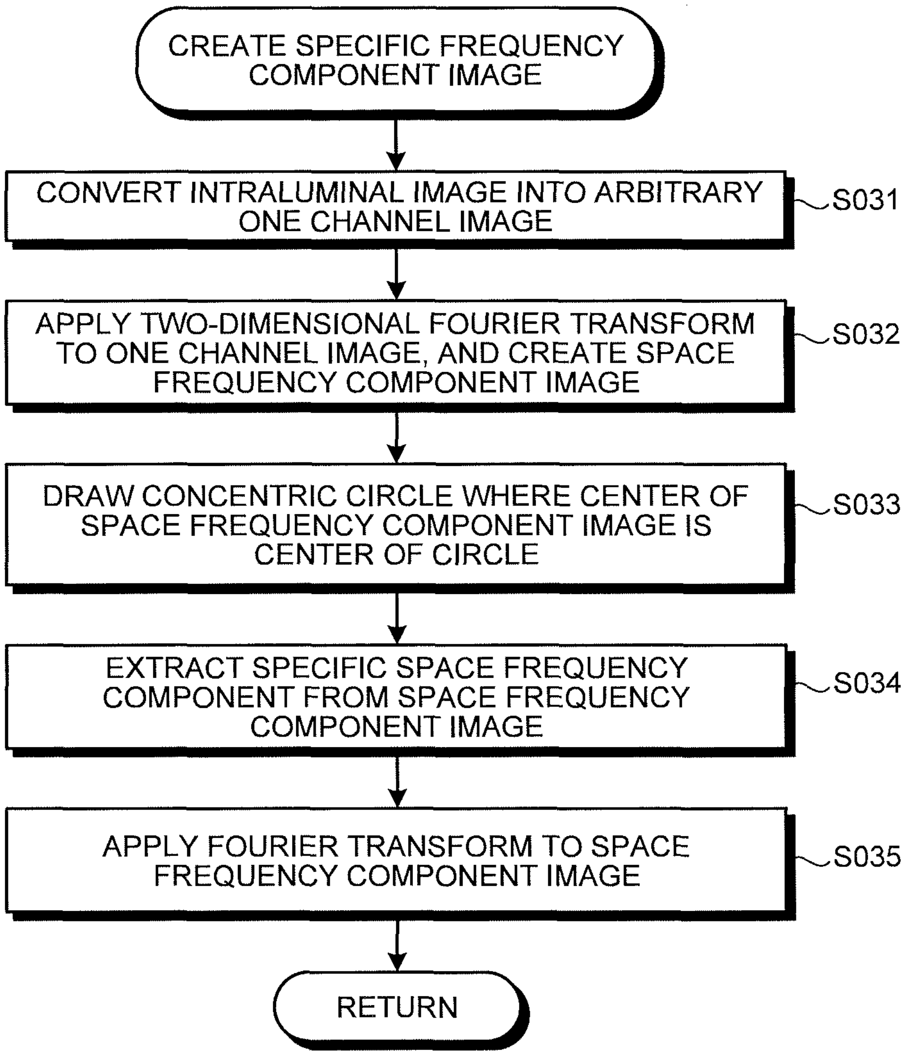
CREATE SPECIFIC FREQUENCY COMPONENT IMAGE
CONVERT INTRALUMINAL IMAGE INTO ARBITRARY ONE CHANNEL IMAGE
S031
APPLY TWO-DIMENSIONAL FOURIER TRANSFORM TO ONE CHANNEL IMAGE, AND CREATE SPACE FREQUENCY COMPONENT IMAGE
S032
DRAW CONCENTRIC CIRCLE WHERE CENTER OF SPACE FREQUENCY COMPONENT IMAGE IS CENTER OF CIRCLE
S033
EXTRACT SPECIFIC SPACE FREQUENCY COMPONENT FROM SPACE FREQUENCY COMPONENT IMAGE
S034
APPLY FOURIER TRANSFORM TO SPACE FREQUENCY COMPONENT IMAGE
S035
RETURN

FIG.6

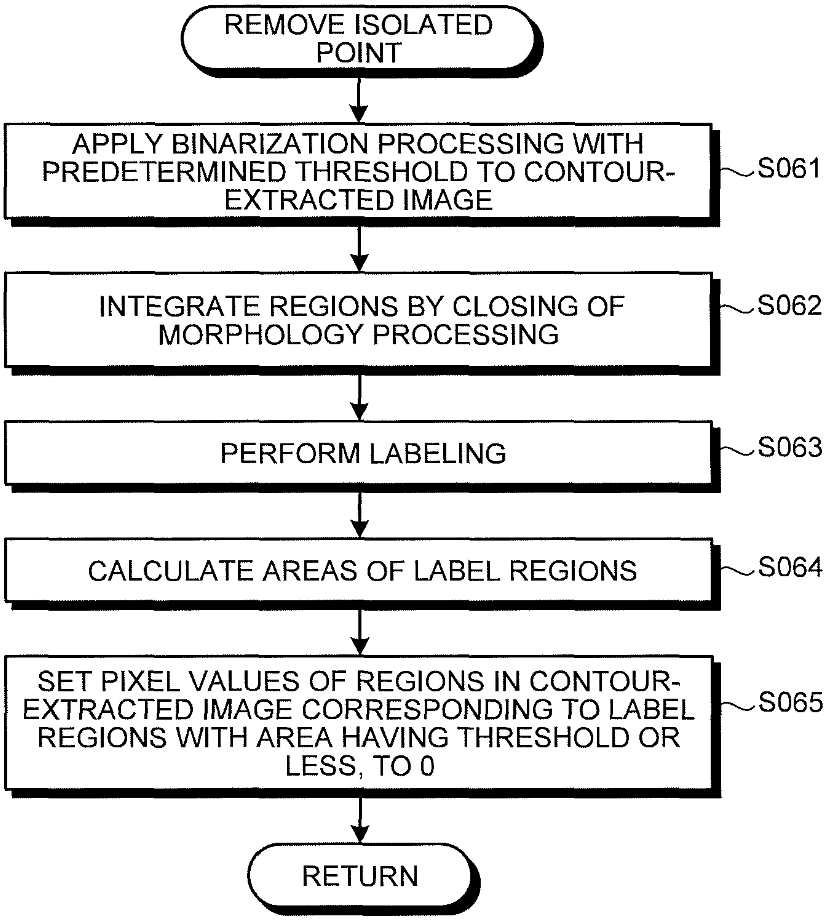
REMOVE ISOLATED POINT
APPLY BINARIZATION PROCESSING WITH PREDETERMINED THRESHOLD TO CONTOUR-EXTRACTED IMAGE
S061
INTEGRATE REGIONS BY CLOSING OF MORPHOLOGY PROCESSING
S062
PERFORM LABELING
S063
CALCULATE AREAS OF LABEL REGIONS
S064
SET PIXEL VALUES OF REGIONS IN CONTOUR-EXTRACTED IMAGE CORRESPONDING TO LABEL REGIONS WITH AREA HAVING THRESHOLD OR LESS, TO 0
S065
RETURN

FIG.7

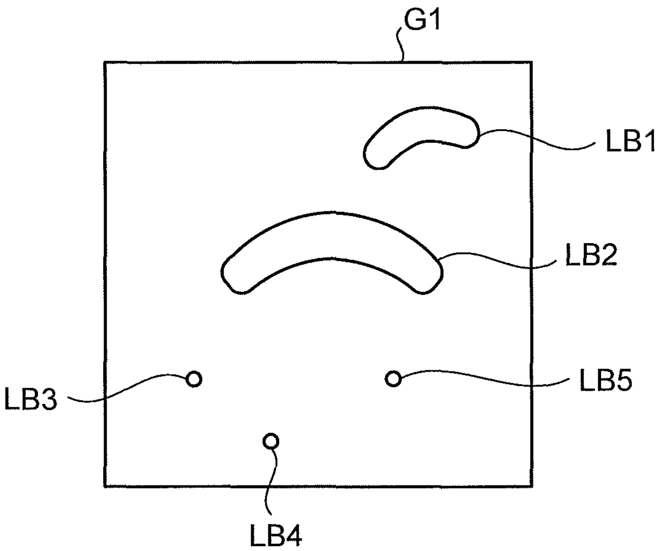
G1
LB1
LB2
LB3
LB5
LB4

FIG.8

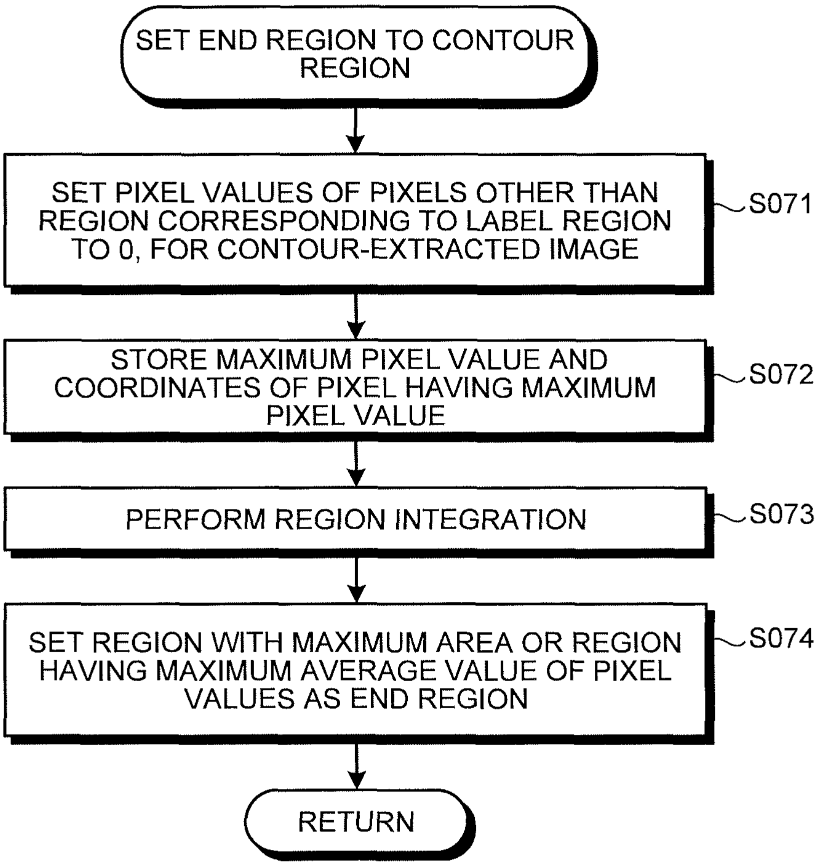
SET END REGION TO CONTOUR REGION
SET PIXEL VALUES OF PIXELS OTHER THAN REGION CORRESPONDING TO LABEL REGION TO 0, FOR CONTOUR-EXTRACTED IMAGE
S071
STORE MAXIMUM PIXEL VALUE AND COORDINATES OF PIXEL HAVING MAXIMUM PIXEL VALUE
S072
PERFORM REGION INTEGRATION
S073
SET REGION WITH MAXIMUM AREA OR REGION HAVING MAXIMUM AVERAGE VALUE OF PIXEL VALUES AS END REGION
S074
RETURN

FIG.9

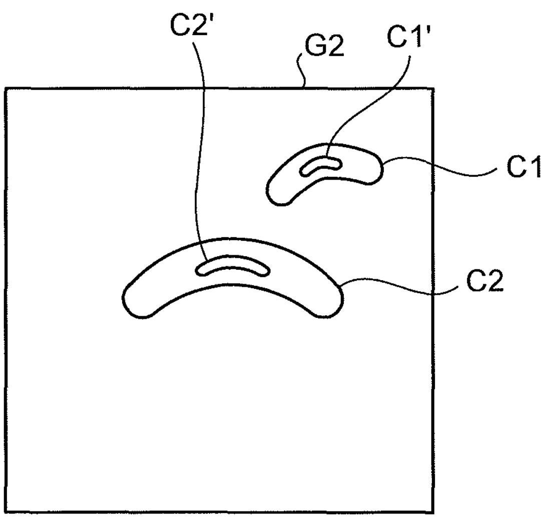
C2'
G2
C1'
C1
C2

FIG.10

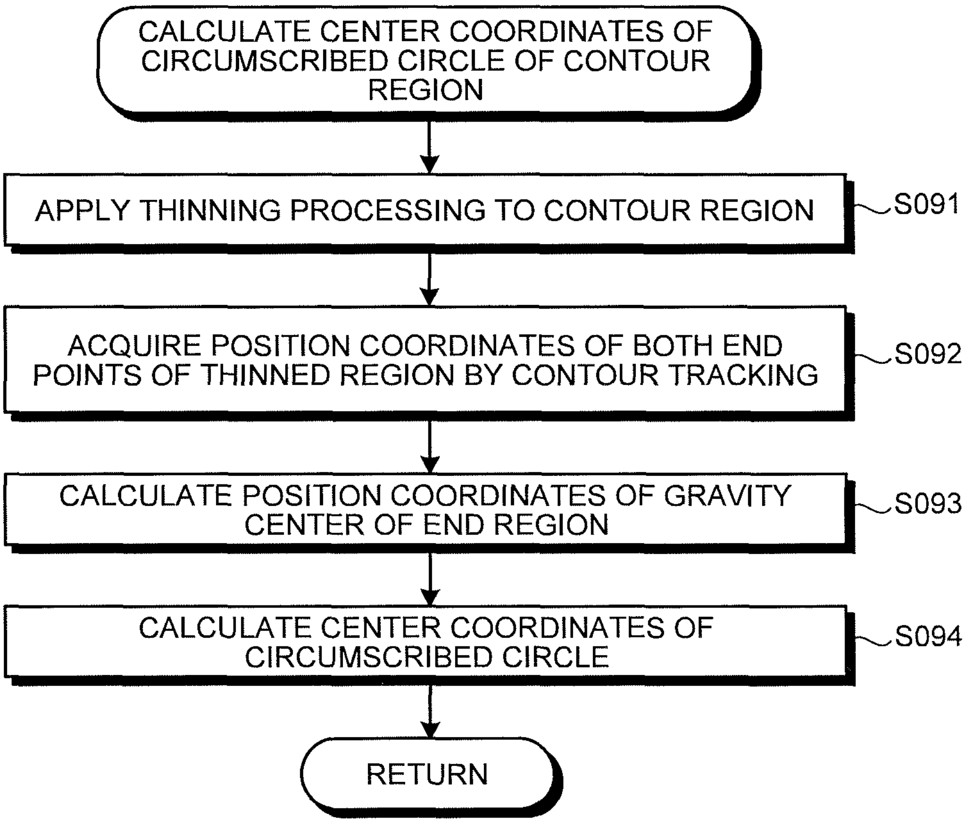
CALCULATE CENTER COORDINATES OF CIRCUMSCRIBED CIRCLE OF CONTOUR REGION
APPLY THINNING PROCESSING TO CONTOUR REGION
S091
ACQUIRE POSITION COORDINATES OF BOTH END POINTS OF THINNED REGION BY CONTOUR TRACKING
S092
CALCULATE POSITION COORDINATES OF GRAVITY CENTER OF END REGION
S093
CALCULATE CENTER COORDINATES OF CIRCUMSCRIBED CIRCLE
S094
RETURN

FIG.11

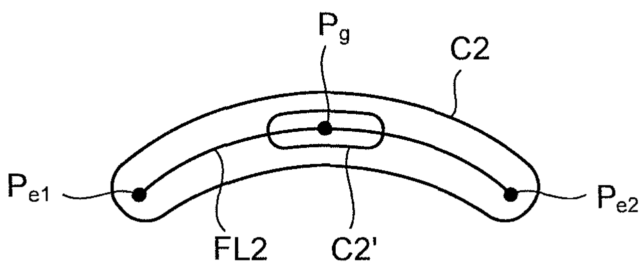
Pg
C2
Pe1
Pe2
FL2
C2'

FIG.12

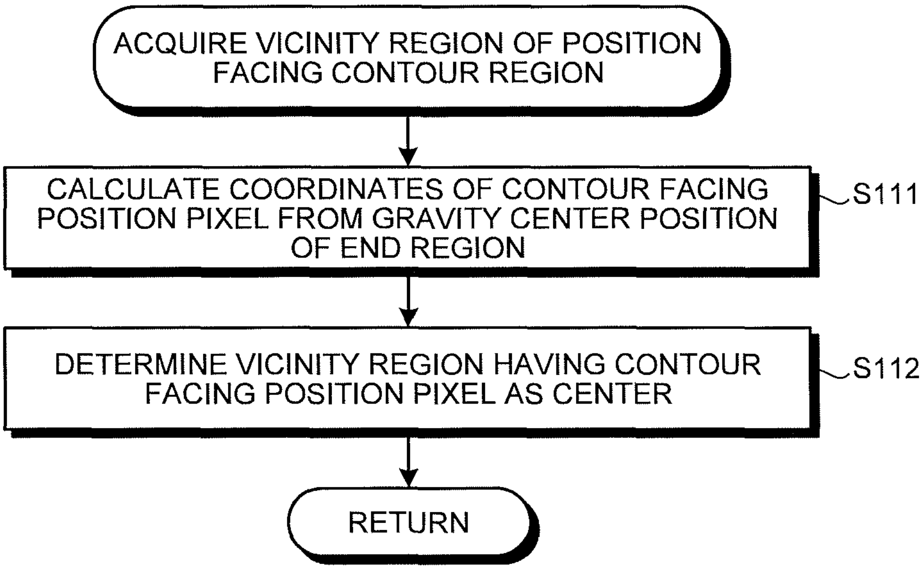
ACQUIRE VICINITY REGION OF POSITION FACING CONTOUR REGION
CALCULATE COORDINATES OF CONTOUR FACING POSITION PIXEL FROM GRAVITY CENTER POSITION OF END REGION
S111
DETERMINE VICINITY REGION HAVING CONTOUR FACING POSITION PIXEL AS CENTER
S112
RETURN

FIG.13

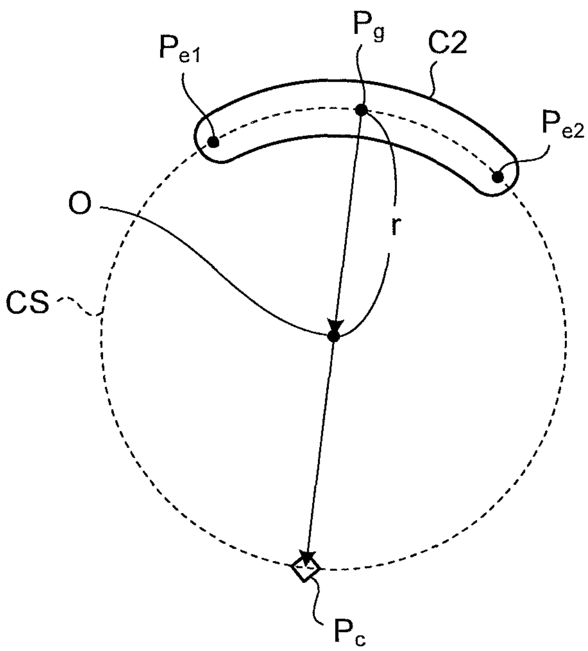
$P_g$
$P_{e1}$
C2
$P_{e2}$
O
r
CS
$P_c$

FIG.14

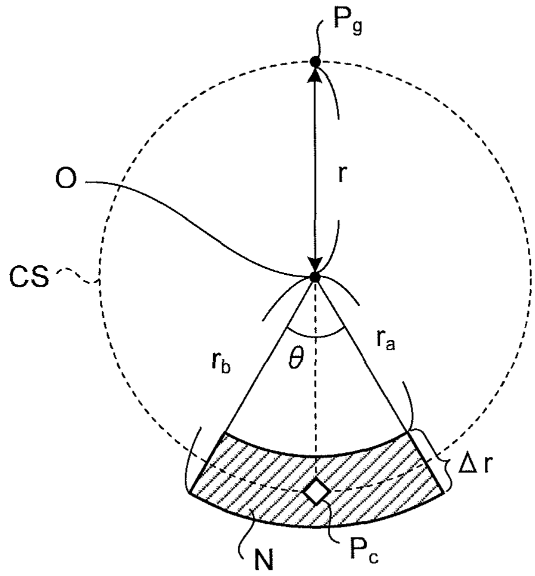
Pg
O
r
CS
rb
θ
ra
Δr
N
Pc

FIG.15

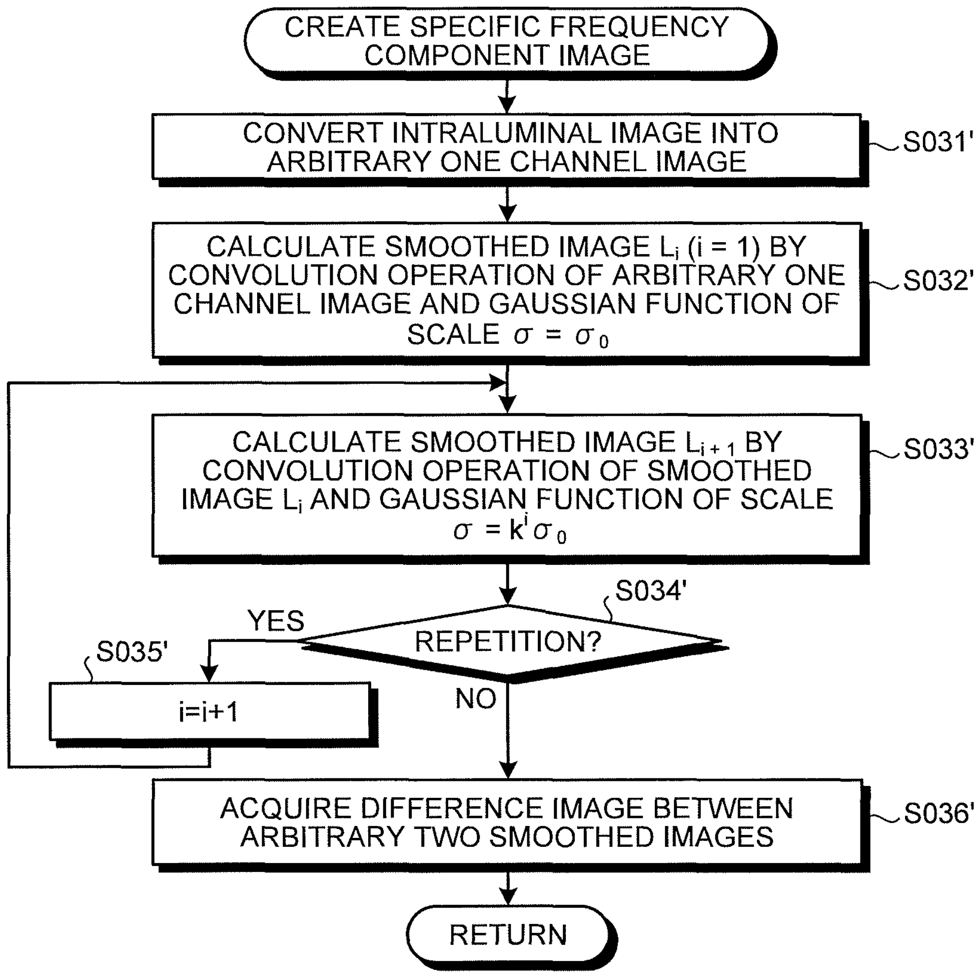
CREATE SPECIFIC FREQUENCY COMPONENT IMAGE
CONVERT INTRALUMINAL IMAGE INTO ARBITRARY ONE CHANNEL IMAGE
S031'
CALCULATE SMOOTHED IMAGE Li (i = 1) BY CONVOLUTION OPERATION OF ARBITRARY ONE CHANNEL IMAGE AND GAUSSIAN FUNCTION OF SCALE σ = σ0
S032'
CALCULATE SMOOTHED IMAGE Li+1 BY CONVOLUTION OPERATION OF SMOOTHED IMAGE Li AND GAUSSIAN FUNCTION OF SCALE σ = k^i σ0
S033'
S034'
REPETITION?
YES
S035'
i=i+1
NO
ACQUIRE DIFFERENCE IMAGE BETWEEN ARBITRARY TWO SMOOTHED IMAGES
S036'
RETURN

FIG.16

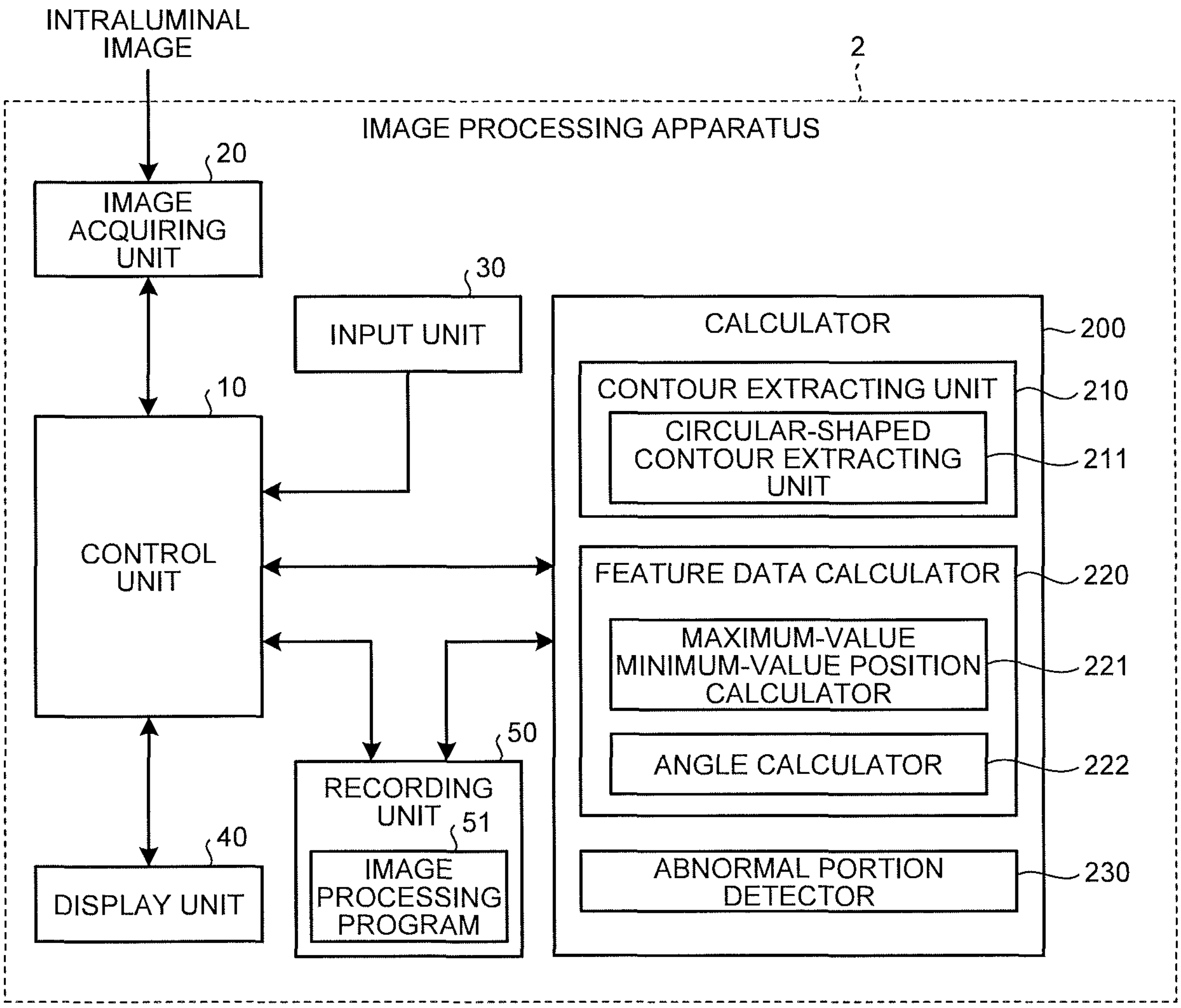
INTRALUMINAL IMAGE
2
IMAGE PROCESSING APPARATUS
20
IMAGE ACQUIRING UNIT
30
INPUT UNIT
CALCULATOR
200
CONTOUR EXTRACTING UNIT
210
CIRCULAR-SHAPED CONTOUR EXTRACTING UNIT
211
10
CONTROL UNIT
FEATURE DATA CALCULATOR
220
MAXIMUM-VALUE MINIMUM-VALUE POSITION CALCULATOR
221
ANGLE CALCULATOR
222
50
RECORDING UNIT
51
IMAGE PROCESSING PROGRAM
40
DISPLAY UNIT
ABNORMAL PORTION DETECTOR
230

FIG.17

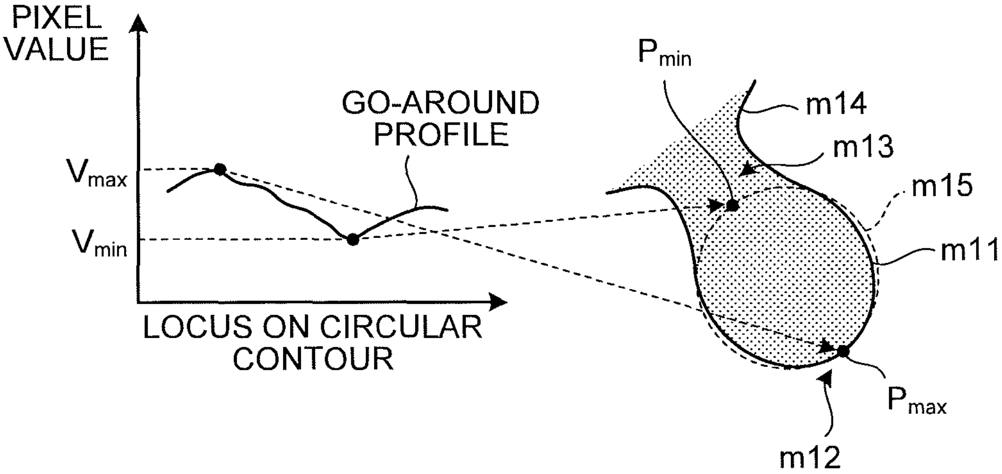
PIXEL VALUE
Vmax
Vmin
GO-AROUND PROFILE
LOCUS ON CIRCULAR CONTOUR
Pmin
m14
m13
m15
m11
Pmax
m12

FIG.18

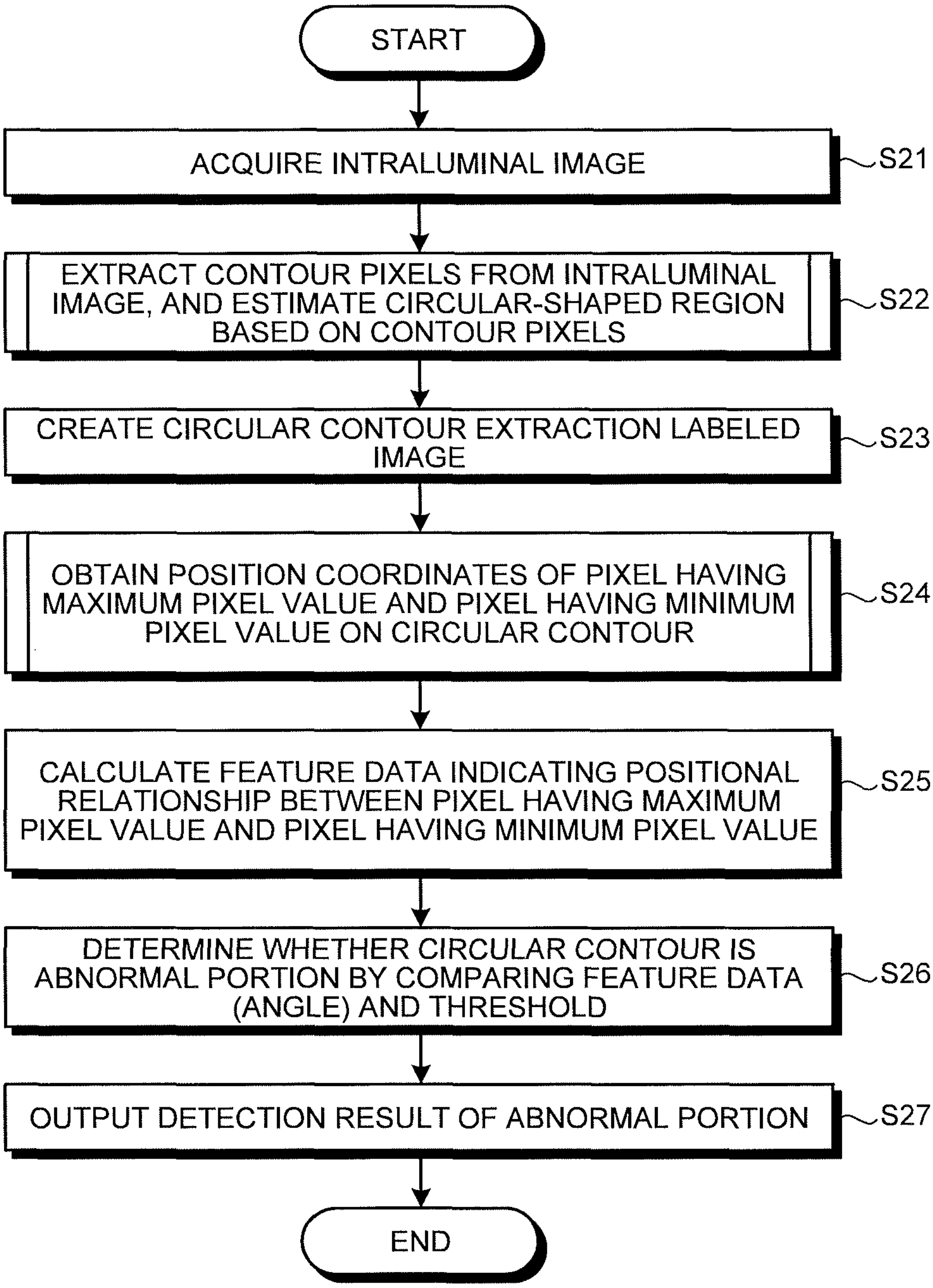
START
ACQUIRE INTRALUMINAL IMAGE
S21
EXTRACT CONTOUR PIXELS FROM INTRALUMINAL IMAGE, AND ESTIMATE CIRCULAR-SHAPED REGION BASED ON CONTOUR PIXELS
S22
CREATE CIRCULAR CONTOUR EXTRACTION LABELED IMAGE
S23
OBTAIN POSITION COORDINATES OF PIXEL HAVING MAXIMUM PIXEL VALUE AND PIXEL HAVING MINIMUM PIXEL VALUE ON CIRCULAR CONTOUR
S24
CALCULATE FEATURE DATA INDICATING POSITIONAL RELATIONSHIP BETWEEN PIXEL HAVING MAXIMUM PIXEL VALUE AND PIXEL HAVING MINIMUM PIXEL VALUE
S25
DETERMINE WHETHER CIRCULAR CONTOUR IS ABNORMAL PORTION BY COMPARING FEATURE DATA (ANGLE) AND THRESHOLD
S26
OUTPUT DETECTION RESULT OF ABNORMAL PORTION
S27
END

FIG.19

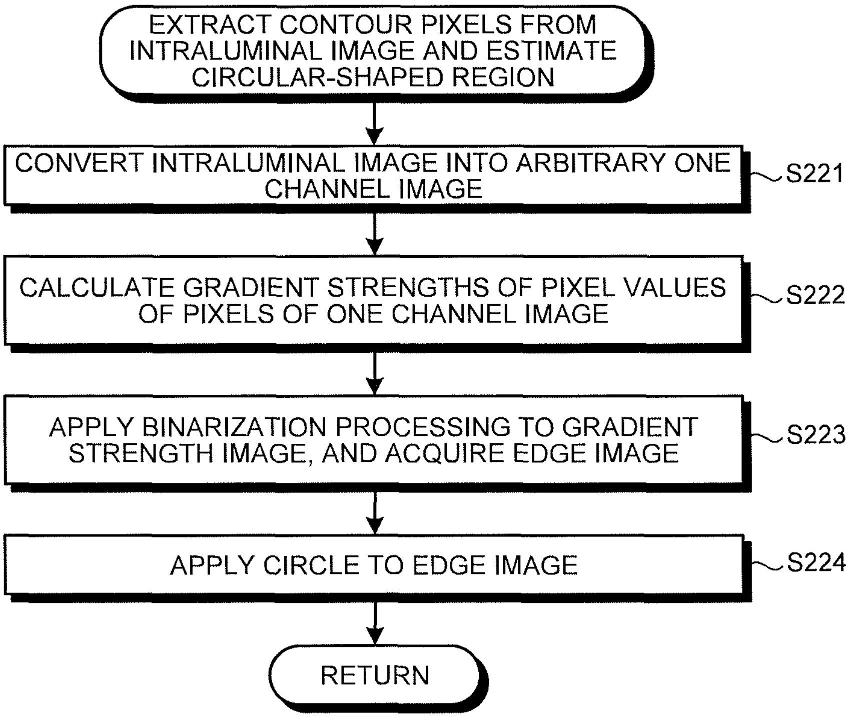
EXTRACT CONTOUR PIXELS FROM INTRALUMINAL IMAGE AND ESTIMATE CIRCULAR-SHAPED REGION
CONVERT INTRALUMINAL IMAGE INTO ARBITRARY ONE CHANNEL IMAGE
S221
CALCULATE GRADIENT STRENGTHS OF PIXEL VALUES OF PIXELS OF ONE CHANNEL IMAGE
S222
APPLY BINARIZATION PROCESSING TO GRADIENT STRENGTH IMAGE, AND ACQUIRE EDGE IMAGE
S223
APPLY CIRCLE TO EDGE IMAGE
S224
RETURN

FIG.20

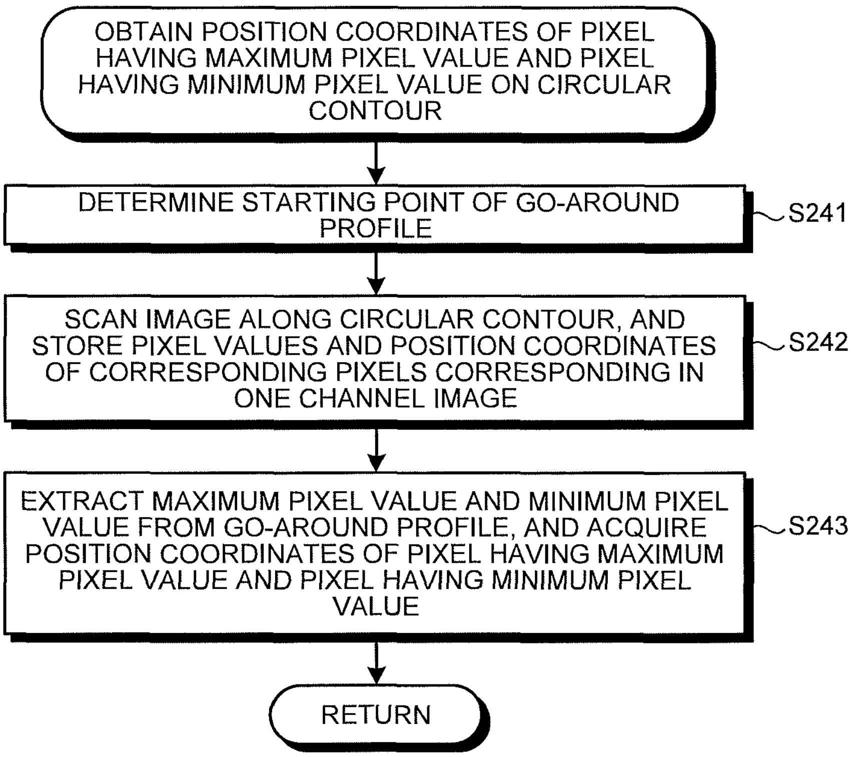
OBTAIN POSITION COORDINATES OF PIXEL HAVING MAXIMUM PIXEL VALUE AND PIXEL HAVING MINIMUM PIXEL VALUE ON CIRCULAR CONTOUR
DETERMINE STARTING POINT OF GO-AROUND PROFILE
S241
SCAN IMAGE ALONG CIRCULAR CONTOUR, AND STORE PIXEL VALUES AND POSITION COORDINATES OF CORRESPONDING PIXELS CORRESPONDING IN ONE CHANNEL IMAGE
S242
EXTRACT MAXIMUM PIXEL VALUE AND MINIMUM PIXEL VALUE FROM GO-AROUND PROFILE, AND ACQUIRE POSITION COORDINATES OF PIXEL HAVING MAXIMUM PIXEL VALUE AND PIXEL HAVING MINIMUM PIXEL VALUE
S243
RETURN

FIG.21

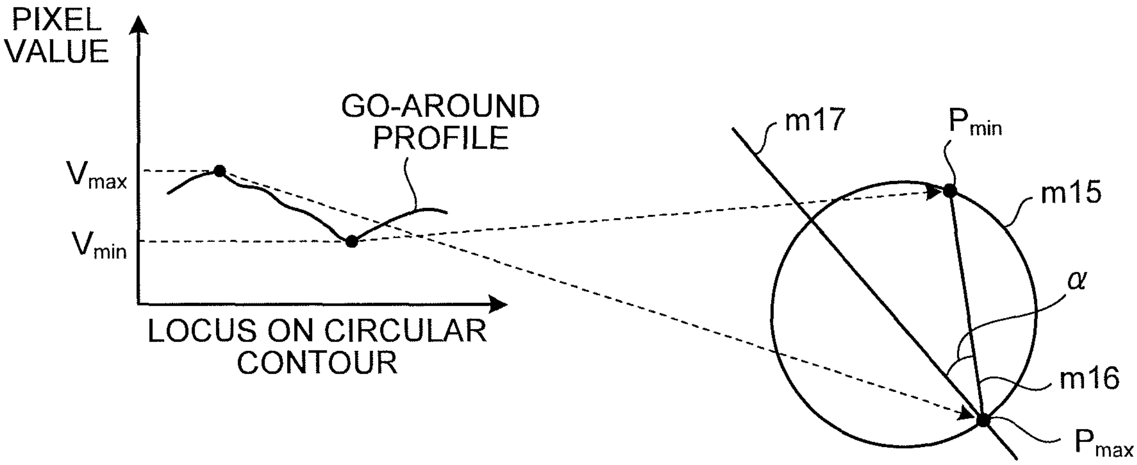
PIXEL VALUE
GO-AROUND PROFILE
Vmax
Vmin
LOCUS ON CIRCULAR CONTOUR
m17
Pmin
m15
α
m16
Pmax

FIG.22

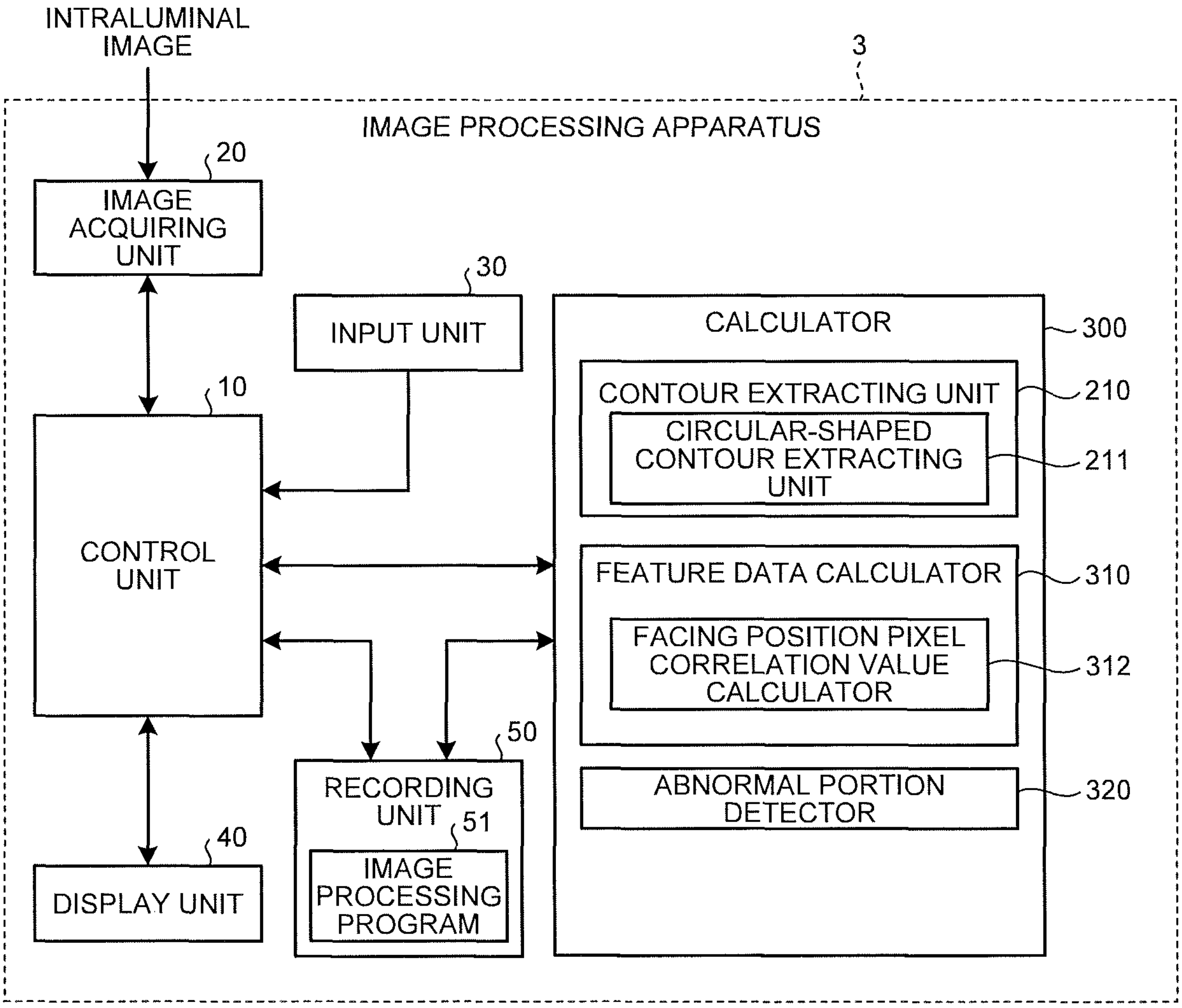
INTRALUMINAL IMAGE
3
IMAGE PROCESSING APPARATUS
20
IMAGE ACQUIRING UNIT
30
INPUT UNIT
CALCULATOR
300
CONTOUR EXTRACTING UNIT
210
CIRCULAR-SHAPED CONTOUR EXTRACTING UNIT
211
10
CONTROL UNIT
FEATURE DATA CALCULATOR
310
FACING POSITION PIXEL CORRELATION VALUE CALCULATOR
312
50
RECORDING UNIT
51
IMAGE PROCESSING PROGRAM
ABNORMAL PORTION DETECTOR
320
40
DISPLAY UNIT

FIG.23

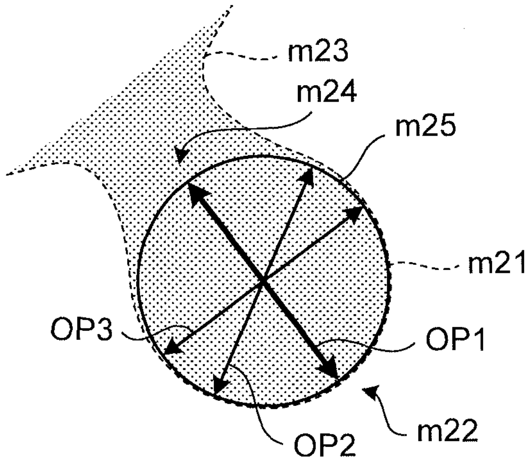


FIG.24

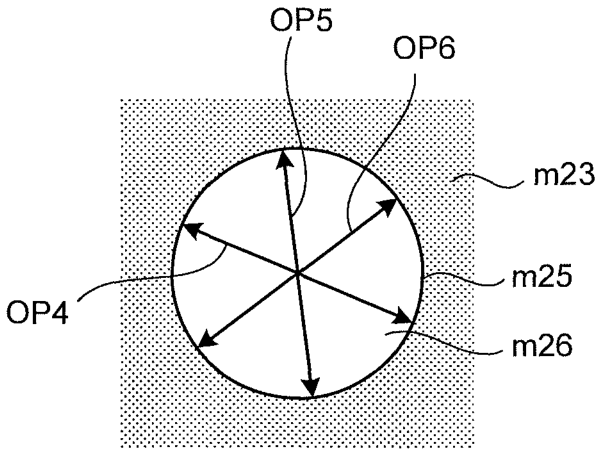

FIG.25

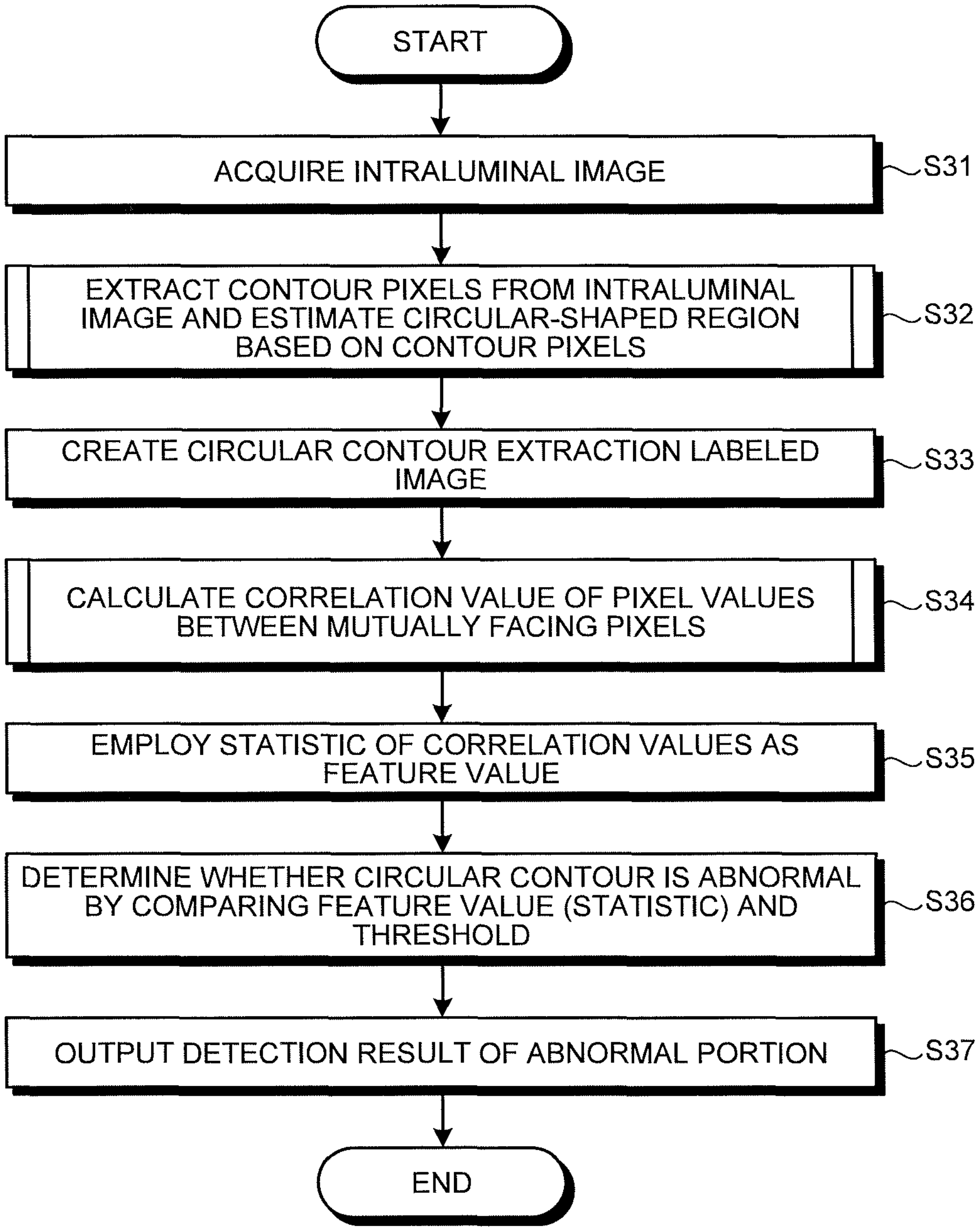
START
ACQUIRE INTRALUMINAL IMAGE
S31
EXTRACT CONTOUR PIXELS FROM INTRALUMINAL IMAGE AND ESTIMATE CIRCULAR-SHAPED REGION BASED ON CONTOUR PIXELS
S32
CREATE CIRCULAR CONTOUR EXTRACTION LABELED IMAGE
S33
CALCULATE CORRELATION VALUE OF PIXEL VALUES BETWEEN MUTUALLY FACING PIXELS
S34
EMPLOY STATISTIC OF CORRELATION VALUES AS FEATURE VALUE
S35
DETERMINE WHETHER CIRCULAR CONTOUR IS ABNORMAL BY COMPARING FEATURE VALUE (STATISTIC) AND THRESHOLD
S36
OUTPUT DETECTION RESULT OF ABNORMAL PORTION
S37
END

FIG.26

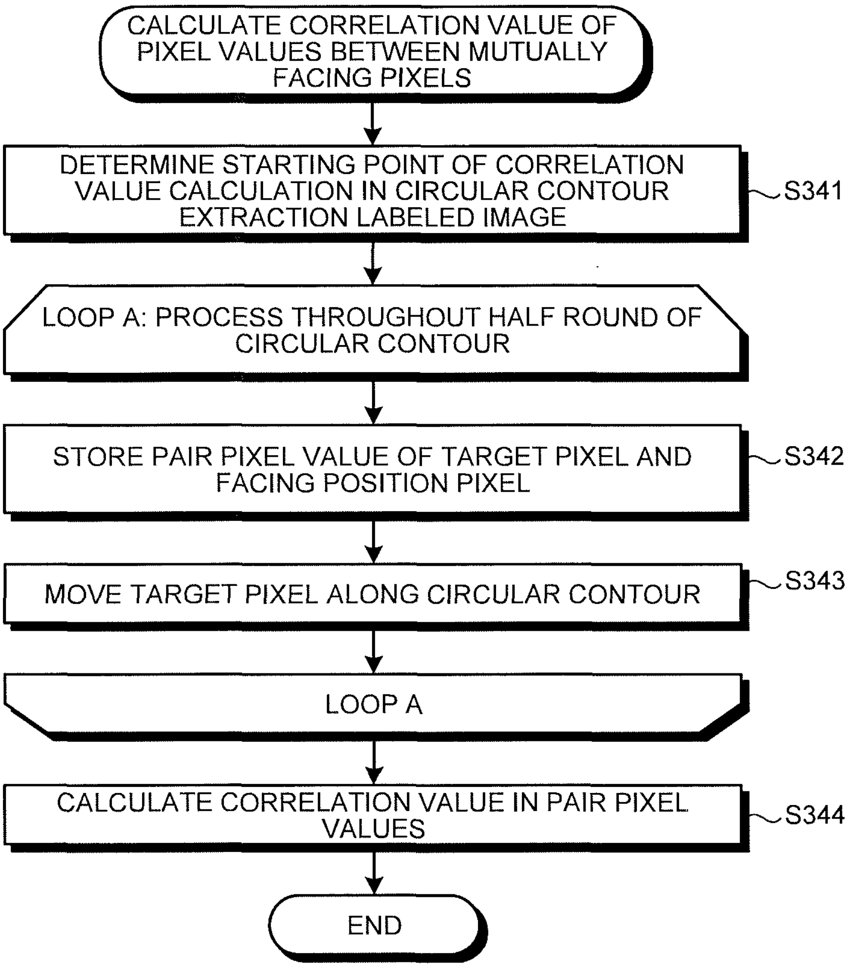
CALCULATE CORRELATION VALUE OF PIXEL VALUES BETWEEN MUTUALLY FACING PIXELS
DETERMINE STARTING POINT OF CORRELATION VALUE CALCULATION IN CIRCULAR CONTOUR EXTRACTION LABELED IMAGE
S341
LOOP A: PROCESS THROUGHOUT HALF ROUND OF CIRCULAR CONTOUR
STORE PAIR PIXEL VALUE OF TARGET PIXEL AND FACING POSITION PIXEL
S342
MOVE TARGET PIXEL ALONG CIRCULAR CONTOUR
S343
LOOP A
CALCULATE CORRELATION VALUE IN PAIR PIXEL VALUES
S344
END

FIG.27

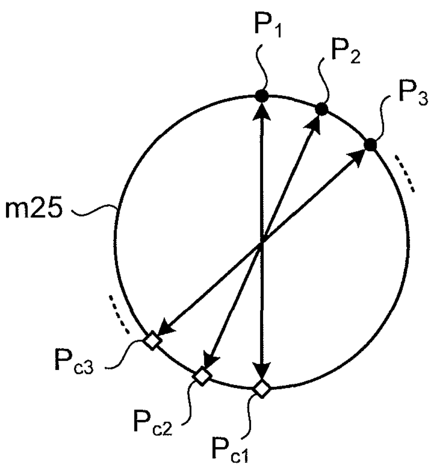
P1
P2
P3
m25
Pc3
Pc2
Pc1

FIG.28

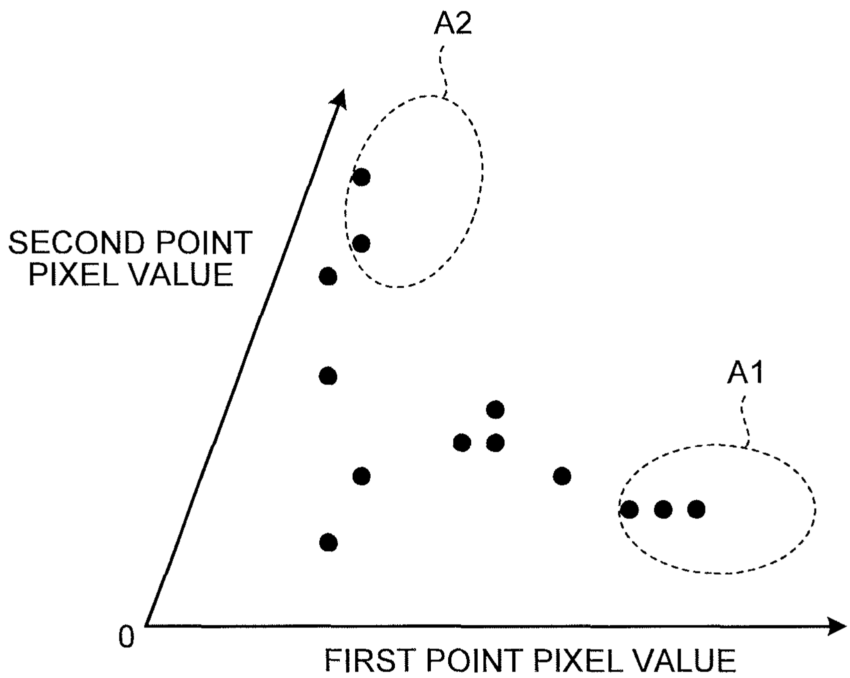

## INTERNATIONAL SEARCH REPORT

International application No.
PCT/JP2013/074903

A. CLASSIFICATION OF SUBJECT MATTER
*A61B1/04*(2006.01)i

According to International Patent Classification (IPC) or to both national classification and IPC

B. FIELDS SEARCHED

Minimum documentation searched (classification system followed by classification symbols)
A61B1/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2009-297450 A (Olympus Corp.),<br>24 December 2009 (24.12.2009),<br>paragraphs [0030], [0055]<br>& US 2011/0085717 A1 & EP 2305091 A1<br>& WO 2009/154125 A1 & CN 102065744 A | 1,7,12<br>2-6,8-10 |
| Y<br>A | JP 2007-244518 A (Olympus Medical Systems Corp.),<br>27 September 2007 (27.09.2007),<br>paragraphs [0062] to [0075]; fig. 5 to 7, 17 to 29<br>& US 2009/0074268 A1 & EP 1994879 A1<br>& WO 2007/105516 A1 & CN 101384211 A | 1,7,12<br>2-6,8-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 October, 2013 (31.10.13) | 12 November, 2013 (12.11.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No. PCT/JP2013/074903

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2007-244519 A (Olympus Medical Systems Corp.),<br>27 September 2007 (27.09.2007),<br>paragraphs [0067] to [0075]; fig. 17 to 20<br>& US 2009/0074270 A1 & EP 1994880 A1<br>& EP 2472473 A1 & WO 2007/105517 A1<br>& CN 101384210 A | 1,7,12<br>2-6,8-10 |
| Y<br>A | WO 2007/119297 A1 (Olympus Medical Systems Corp.),<br>25 October 2007 (25.10.2007),<br>paragraphs [0121] to [0122]<br>& US 2009/0074269 A1 & EP 1994878 A1<br>& CN 101404923 A | 1,7,12<br>2-6,8-10 |
| A | JP 2007-244517 A (Olympus Medical Systems Corp.),<br>27 September 2007 (27.09.2007),<br>paragraph [0047]; fig. 15<br>& US 2009/0073257 A1 & EP 1994876 A1<br>& WO 2007/119295 A1 & CN 101389260 A | 1-10,12 |

Form PCT/ISA/210 (continuation of second sheet) (July 2009)

**INTERNATIONAL SEARCH REPORT**

International application No.
PCT/JP2013/074903

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 11
because they relate to subject matter not required to be searched by this Authority, namely:
(See extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2013/074903

Continuation of Box No.II-1 of continuation of first sheet(2)

The invention of an image processing method according to claim 11 can include: how to process an image while a doctor is using a device (an endoscope) in the human body; and a step of determining a condition of the human body, such as a human disease or health condition, for a medical purpose of "detecting an abnormal part." The invention therefore pertains to a surgical operation and a diagnostic method for the human body, and thus relates to a subject matter on which the International Preliminary Examining Authority is not required to carry out an international preliminary examination under the provisions of PCT Article 17(2)(a)(i) and PCT Rule 39.1(iv).

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005192880 A
- JP 2918162 B
- JP 2002165757 A


### Non-patent literature cited in the description


- Digital Image Processing. CG-ARTS Association, 114-117
- **CORONA PUBLISHING CO., LTD.** Morphology. 82-90
- Digital Image Processing. CG-ARTS Association, 181-182
- Digital Image Processing. CG-ARTS Association, 185-186
- Digital Image Processing. CG-ARTS Association, 178-179
- Digital Image Processing. CG-ARTS Association, 182-183
- Digital Image Processing. CG-ARTS Association, 114-121
- Digital Image Processing. CG-ARTS Association, 211-214
- Digital Image Processing. Reference: CG-ARTS Association, 197-198
- Digital Image Processing. CG-ARTS Association, 229-230